# EUROPEAN PATENT APPLICATION

(11) **EP 1 621 630 A1**
(43) Date of publication of application: **01.02.2006**
(21) Application number: 04077174.3
(22) Date of filing: 28.07.2004
(51) Int. Cl.: C12N 15/82, C07K 14/415, C12N 5/00

(54) **Method for preventing dehiscence and altering plant lignification**

(71) Applicant: Plant Research International B.V., 6708 PB Wageningen (NL)
(72) Inventor: Aharoni, Asaph, Tel-Aviv (IL); Pereira, Andy, 6716 GG Ede (NL)
(74) Representative: Winckels, Johannes Hubertus F.

(57) **Abstract**

The invention relates to a method to prevent dehiscence in a plant or to alter plant lignification comprising providing said plant with a nucleotide protein coding for a GGL protein or a functional equivalent thereof, and growing said plant. The invention also relates to the isolated GGL protein or functional equivalents, a nucleic acid encoding said protein and vectors, host cells and plants comprising said nucleic acid.

## Description

The invention relates to a method for preventing dehiscence or pod shatter in silique type fruits and a method to alter lignification of stem tissue.

### Background of the Invention

Many economically important plants bear fruits in pods or siliques, for instance the legumes, like beans and peas and the *Brassicacea,* such as oilseed rape and mustard. The major loss in harvesting the fruits of those plants is due to the early or premature opening of the fruits, whereby the seeds fall out, a phenomenon called pod shatter or dehiscence. It is estimated that 20% loss is due to early pod-shattering in oilseed crop plants.

In the *Brassicaceae* family the pod-shattering process is directly controlled by lignification. *Arabidopsis thaliana,* which is a typical member of more than 3,000 species of *Brassicaceae,* produces dry, dehiscent fruit in which two carpel valves are joined to the replum, a thin structure that separates the valves. Dehiscence is a carefully orchestrated event that assists in the dispersal of the seeds. This process requires the prior formation of the dehiscence zone, a region no more than a few cells wide that extends the entire length of the fruit at the valve-replum boundaries. The dehiscence zone consists of a cell separation layer as well as an adjacent region of lignified cells. The actual opening of the fruits is caused by the tension that is created in the silique around the valve margins by localized lignification and cell wall hydrolysis in specialized areas.

Changes in levels, time and place of lignification may possibly lead to mutant plants which have aberrant pod shattering. In most cases the differences between such "shattering" mutants and normal wild-type plants would be easily recognized when touching a mature, dry silique.

Considering the economic importance of pod-shattering there is a great need for controlling this phenomenon.

More than a dozen genes have been identified in *Arabidopsis* which function in silique formation and development. Several of them belong to the MADS-box gene family which in the case of *Arabidposis* is known to contain more than 40 members with diverse expression patterns, and play a wide variety of roles during plant development. Most of the plant members of this family contain four domains, referred to as MIKC-motifs. The MADS-domain (M) itself is a highly conserved DNA-binding motif, and is followed by a highly divergent intervening (I) region. The K-domain is similar to the coiled-coil domain of keratin, followed by the C-domain that is probably involved in transcriptional activation. The I- and K-domains are likely to be involved in dimerization; MADS-box gene products are known to form either homo- or heterodimers.

One of the genes that seems to be at the base of all floral development processes is the MADS-box gene *AGAMOUS (AG).* The α*g* mutant produces flowers with no carpels but with an indeterminate reiteration of the pattern (sepals, petals, petals)ₙ. Thus, *AG* is required for the identity of carpels and stamens, and seems to act upstream of all below-mentioned genes. The fact that *AG* expression is maintained throughout stamen and carpel development suggests that, in addition to its role in specifying the identity of those organs, *AG* may be important for the differentiation of specific cell types later in flower/seed development.

The MADS-box genes *SHATTERPROOF1 (SHP1)* and *SHATTERPROOF2 (SHP2)* (also known as *AGL1* and *AGL5)* are two genes that specify the valve margins of the siliques. They promote lignification of the valve margin adjacent to the dehiscence zone, enabling the silique to dehisce (Liljegren et al 2000 Nature 404: 766-770). When both these genes are knocked out, no dehiscence zone is formed and no lignification takes place in the valve margin. When they are ectopically (over-)expressed, the whole valve gets a valve margin identity, resulting in ectopic lignification and thinner siliques than usual, which in turn causes seed crowding and large tears over the length of the silique.

*FRUITFULL (FUL),* another MADS-box gene (also known as *AGL8)* is usually expressed in the floral shoot apex, young flower primordial, developing carpels and finally in the carpel valves. It promotes differentiation and expansion of the valves. When it is knocked out, siliques no longer elongate after fertilization, due to the fact that the valve cells fail to differentiate, which leads to seed crowding and valve rupture (Ferrándiz et al 2000 Science 289: 436-438). Furthermore, in the *ful* knock-out mutant the whole inner valve gets lignified, as if the valves get a replum identity, and as a result siliques fail to dehisce. When *FUL* is ectopically (over-)expressed, no dehiscence zone forms at all, neither is there any lignification adjacent to the valve margin and both valve margin and replum cells seem to become valve cells, resulting in one continuous big valve without boundaries. This also leads to reduced pod-shattering. It appears that *FUL* is expressed in the valves only, which indicates a repressor role of the gene. Indeed the *FUL* gene is a negative spatial regulator of *SHP1*/*2* and thus prevents extopic lignification, ensuring that only the cells in the valve margin get the valve margin cell fate. *SHP* and *FUL* have complementary expression patterns and are antagonists. This is also apparent from the fact that *FUL* overexpression results in a similar phenotype as *shp1* / *shp2* knock-out (indehiscence due to lack of lignification), and in turn *SHP1*/*2* overexpression results in a similar phenotype as *ful* knock-out (seed-crowding and ectopic lignification).

GT140 is a so-called valve margin molecular marker, showing very specialized expression only in the valve margins of the silique. This marker corresponds to a bHLH transcription factor, *INDEHISCENT (IND1),* which is required for dehiscence zone differentiation (Liljegren et al, 2004, Cell 116: 843-853). Loss-of-function *ind1* mutant siliques lack the lignified patches at the valve margin and are unable to shatter, and *IND1* ectopic expression in *ful* mutant valves appears to be responsible for most of the *ful* phenotypes in the fruit. YJ36, another valve margin marker, is expressed on the inner and outer surfaces of the valve margin and in the septum. YJ161 is expressed in the inner and outer epidermis of the valve margin, and corresponds to a putative zinc finger protein. The marker YJ80 is expressed in the valve margin and the seed abscission zone and maps close to a gene which is similar to mammalian ankyrins. The YJ115 insertion is upstream of a gene that is expressed in the abaxial replum and in the valve margins and which encodes for an unknown protein with a putative transmembrane domain.

Another indehiscent mutant, *alcatraz (alc),* owes its phenotype to the fact that it lacks a specific layer of unlignified cells within the dehiscence zone (Rajani and Sundaresan 2001 Curr Biol 11: 1914-1922). The external appearance of the dehiscence zone in *alc* mutants, though, is not detectably different from that of wild-type siliques. All silique structures develop normally in *alc* mutants, including the replum; except that in the later stage of silique ripening there is the formation of a lignified "bridge" at the valve-replum interconnection on the inside of the fruit. This makes the silique non-shattering without many other side-effects. The siliques still can be opened by applying simple pressure on the replum, without requiring breaking of the entire fruit walls. Since silique expansion and lignification proceeds otherwise normally in *alc* siliques, *ALC* must act either downstream of the *SHP* genes or independently in a parallel pathway. GUS staining showed that after fertilization there is expression only at the valve margins, whereas it is diffused throughout the gynoecium before. The affected gene was found to be At5g67110 and it encodes a myc/bHLH-related protein, which belongs to a broad group of proteins that function by binding DNA through their basic domain and dimerizing by means of their helix-loop-helix region (Rajani and Sundaresan 2001, supra).

Breakdown of the cell wall proceeds through the action of extracellular enzymes such as cellulases and pectinases. The first gene cloned that has been demonstrated to be related to pod shattering encodes a polygalacturonase (PG), an enzyme responsible for degradation of the major component of pectin, expressed in the dehiscence zone of *Brassica napus* pods. In addition to transcriptional regulation, a post-transcriptional control of PG activity has been proposed, since it has a cleavable N-terminal domain. The presence of the N-terminal domain appears to block PG secretion in the cell wall, and only upon its removal, triggered by an unknown signal, is the mature protein directed to the extracellular compartment to exert its function.

An increase in β-1,4-glucanase (cellulase) activity has been reported during *Brassica napus* pod dehiscence, although the corresponding gene has yet to be identified. Another enzymatic activity related to cell wall loosening is xyloglucan endotransglycosylase (XET). An XET-encoding gene is specifically up-regulated in the dehiscence zone of *Brassica napus* at the final stages of pod development, and there are indications that it is positively regulated by *SHP1*/*2.*

Many other enzymatic activities are probably related to achieve pod shatter, such as those related to lignin synthesis. However, to date very little is known about the mechanisms regulating these enzymes. Abscission processes have been linked by considerable evidence to the opposing action of the plant hormones ethylene and auxin. Ethylene works by promoting abscission, while auxin acts to delay it. Together, the balance between these hormones appears to be an important factor regulating the timing of abscission. Auxins are likely to play a dominant role in dehiscence regulation. In *Brassica*, an increase in cellulase activity in the dehiscence zone was shown to correlate with a specific decrease in auxin content in those cells. In addition, the exogenous application of auxin analogues to *Brassica* developing pods significantly delayed dehiscence sell separation. It was observed that these auxin analogues inhibited the increase in cellular activity and, interestingly, also affected PG pectinase activity by blocking its secretion to the cell wall. Related to the possible role of auxin in dehiscence regulation, the expression pattern of the bHLH transcription factor *SPATULA (SPT)* is suggestive. *SPT* is required for the development of carpel tissues specialized in pollen tube growth, but SPT mRNA was also found in the dehiscence zone of grown siliques and in the seed abscission zone. Diverse evidence indicated that *SPT* is responsive to auxin: it has been shown that *ETTIN,* an auxin-responsive-factor (ARF) represses *SPT* in some tissues; and inhibitors of auxin polar transport suppress spt carpel phenotypes. SPT might be a link between cell differentiation and auxin related dehiscence zone formation (Ferrándiz, C., 2002, J. Exp. Botany 53(377) 2031-2038).

Lignin is one of the most abundant natural product and is present in plant cell walls of conductive and supporting tissues, such as tracheary elements and fibers. The lignin confers mechanical strength so that these tissues can withstand enormous pressure, e.g. produced during transpiration. Lignin also provides the rigidity to wood, resistance to biochemical degradation and may also deter herbivore feeding. It is often induced in response to infection or wounding, providing a physical barrier to pathogen penetration.

Lignin is a complex phenylpropanoid polymer derived from the dehydrogenative polymerization of monolignols: p-coumaryl alcohol, coniferyl alcohol and synapyl alcohol. The heterogeneity of lignin is due to the different proportions of these three monolignols in different plant species, developmental stages and tissue types. Lignin heterogeneity is likely to be controlled by the differential expression of lignin enzymes in different lignified tissues. The guaiacyl (coniferyl-derived) lignins are characteristic of softwoods in gymnosperms (pines), whereas angiosperm dicot lignin is primarily composed of guaiacyl and syringyl (synapyl-derived) units like in hardwoods such as oak, and monocot grass lignin is a mixture of guaiacyl, syringyl and p-hydroxylphenyl (coumaryl-derived) units. The ratios of guaiacyl to syringyl lignin differ in woods and contribute to their differences in chemical degradation during industrial pulping, the softwoods being more resistant to degradation than the hardwoods. For industrial use the goal would be to develop plants with increased levels of syringyl lignin to facilitate its chemical degradation. However no plants have been found to contain purely syringyl lignin.

The developmental regulation of lignin deposition in angiosperms, produces primary xylem with guaiacyl lignin, whereas the secondary xylem and sclerenchyma produce guaiacyl-syringyl lignin (Chapple et al., Plant Cell 4, 1413, (1992)). One way to change lignin composition is to knockout or silence the expression of one or more of the enzymes identified in lignin biosynthesis. Another way to regulate the lignin composition would be regulate the secondary lignification pathway by expressing transcription factor genes to change the expression of specific enzymes and thus change lignin composition and increase the syringyl:guaiacyl ratio.

### Summary of the Invention

The present invention now describes a method to prevent dehiscence in a plant comprising providing said plant with a nucleotide sequence GARGOYLE (GGL) coding for a GGL protein or a functional equivalent thereof, and growing said plant. Preferably the GGL protein is overproduced in the replum of a plant, wherein said plant is a member of the *Brassicaceae,* preferably *Brassica napus.*

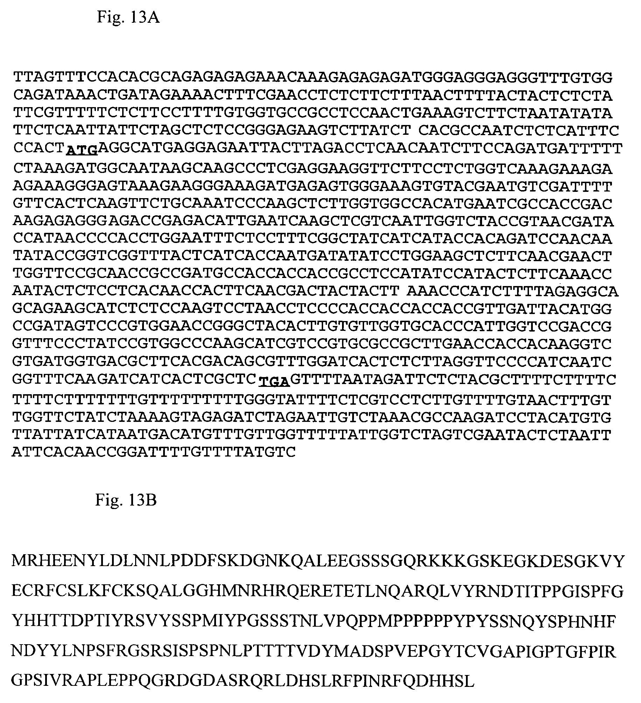

Another embodiment of the invention is a method to alter lignification in a plant providing said plant with a nucleotide protein coding for a GGL protein or a functional equivalent thereof, and growing said plant. The invention also relates to a GGL protein having a sequence comprising the amino acid sequence of Fig. 13B, or a functional fragment thereof. Such a protein is preferentially encoded by the open reading frame contained in the nucleotide sequence of Fig. 13A.
Further, the invention relates to a vector comprising said nucleotide sequence, preferably wherein the nucleotide sequence is operably linked with a replum specific promoter, and a host cell comprising this vector.
Also encompassed in the invention is a plant transformed with such a vector. Finally, the invention relates to the use of a GGL protein, a nucleotide sequence encoding such a protein, a vector comprising such a nucleotide sequence or a host comprising said vector to perform a method according to the invention.

### Legends to the Figures

Figure 1: Phenotype of the *gargoyle (ggl)* mutant showing recumbent stem and elongated leaves (A and B).
Figure 2: Altered lignification in the *ggl* mutant stem.
   Bottom segments of 6 weeks old wild type and *ggl* mutant plants were stained with phloroglucinol (PG) and Toluidine blue 0 (TB). Red staining in the PG stained sections indicates the presence of lignin, while in the TB stained sections all types of cell wall components including lignin are stained blue.

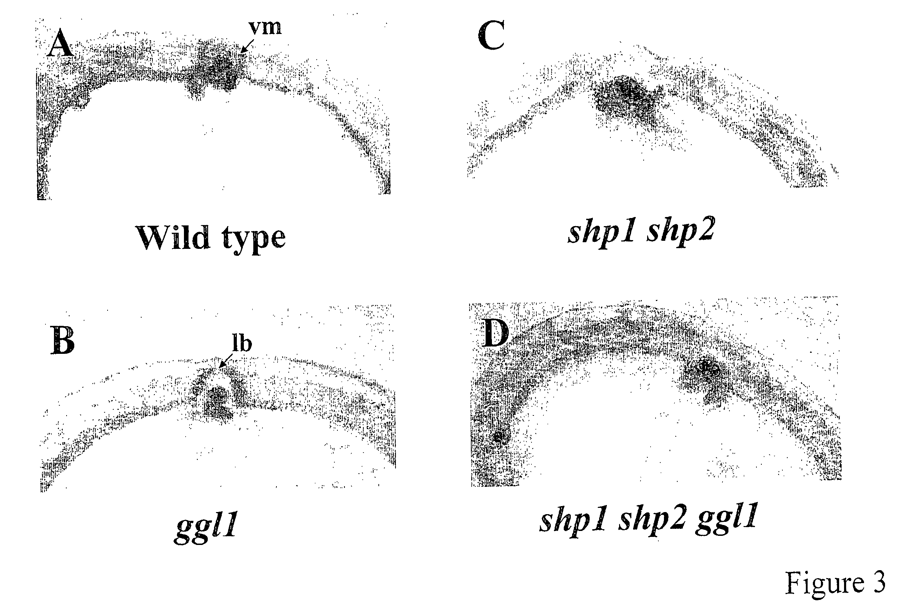

Figure 3: Ectopic lignification in the replum of the *ggl* mutant.
   (A) In wild type fruit valve margins are lignified (vm).
   (B) In *ggl* lignified cells similar to the ones in the valve margins penetrate the replum and this results in a "lignification bridge" (Ib).
   (C) In the *shp1 shp2* double mutant, lignification in the valve margin does not occur.
   (D) In the *shp1 shp2 ggl* triple mutants valve margin cells are not lignified as for the *shp1 shp2* double mutant.

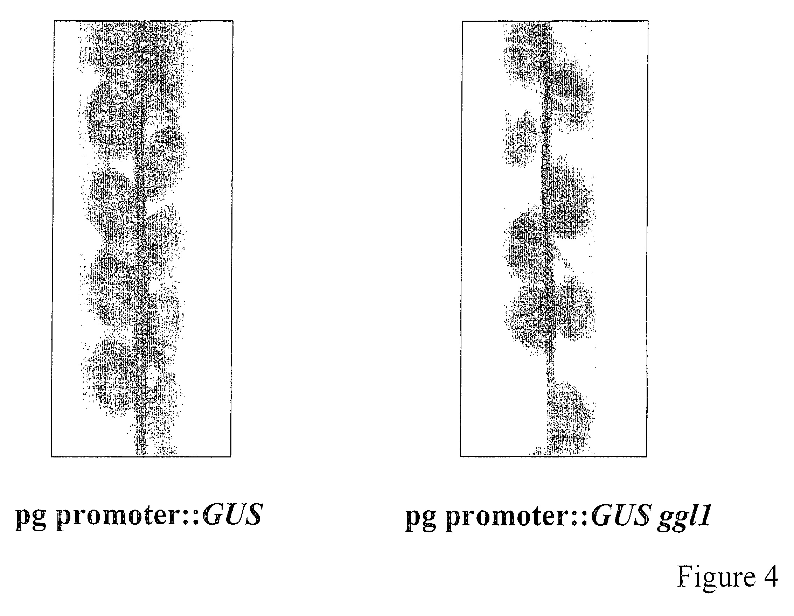

Figure 4: The Polygalacturonase (PG) valve margin marker is expressed in the *ggl* mutant replum.
   The *ggl* mutant was crossed to a PG promoter::GUS line. Expression of the PG molecular marker is in the valve margin while it is ectopically expressed in the replum of the *ggl* mutant.

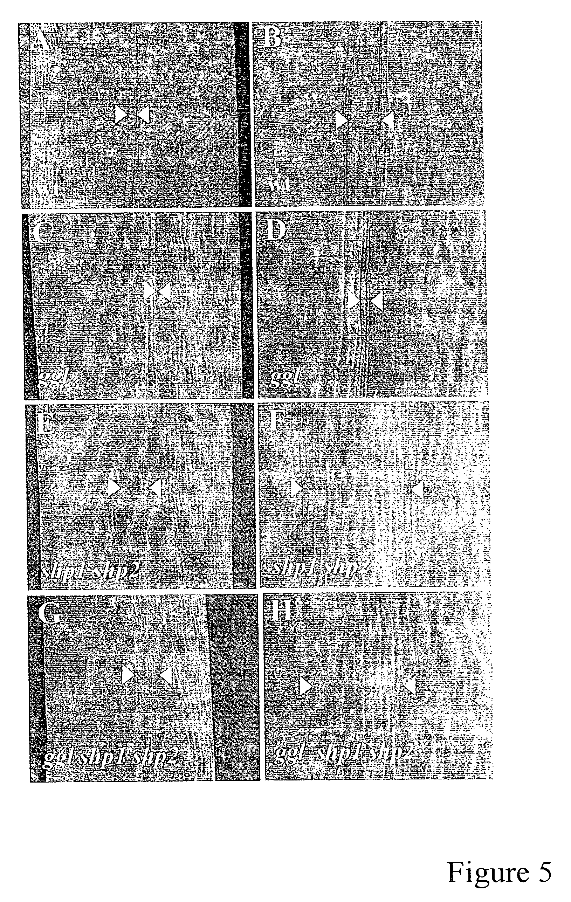

Figure 5: Alteration to replum development in the *ggl* mutant detected by SEM analysis.
   The arrow heads mark the replum region in mature siliques (stage 17) of the different samples.

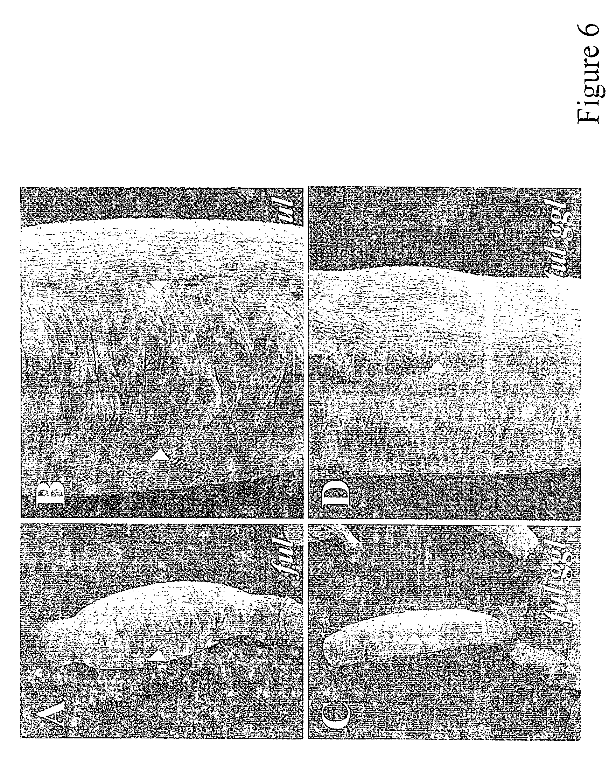

Figure 6: Overexpression of *GGL* restores replum development in the *fruitful (ful)* mutant, detected by scanning electron microscopy (SEM).
   (A) SEM picture of the single *ful* mutant mature silique.
   (B) A close observation of the twisted replum region in the single *ful* mutant.
   (C) SEM picture of the double *ful*/*ggl* double mutant mature silique.
   (D) A close observation of the twisted replum region which is partially restored in the *ful*/*ggl* double mutant.

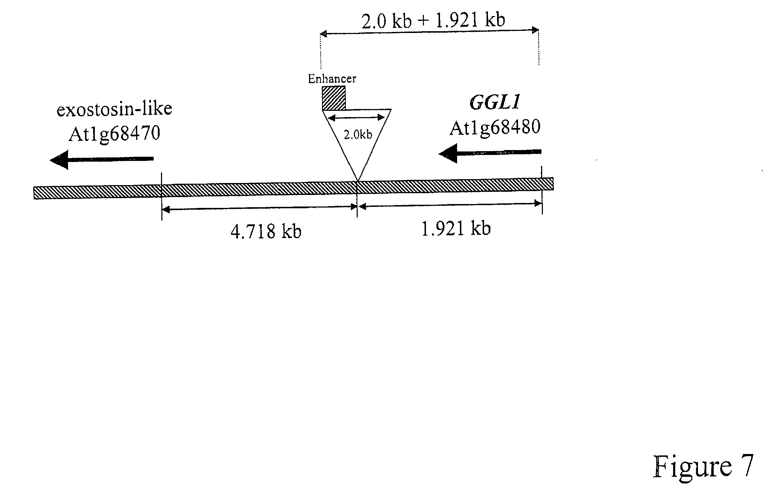

Figure 7: Location of the activation tag insertion in relation to the *GGL1* gene (At1g68480) and the adjacent gene At1g68470 (exostosin-like).

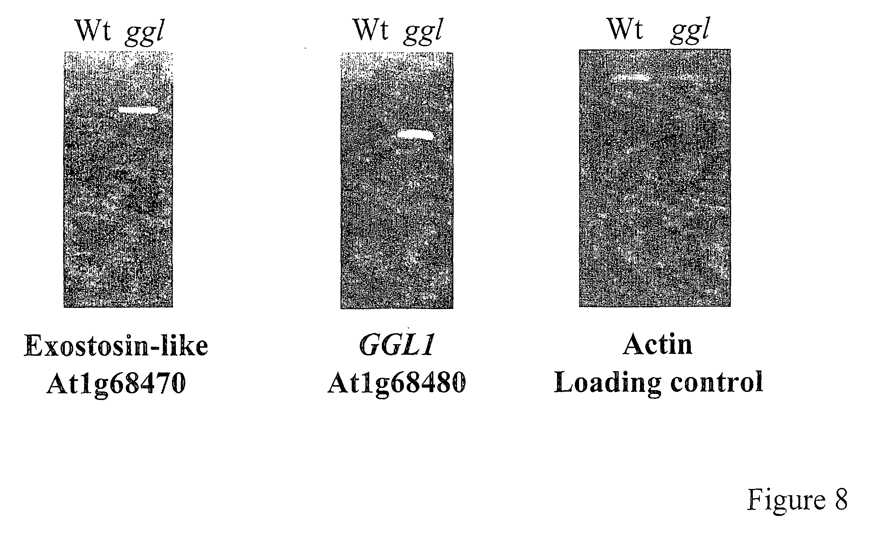

Figure 8: Expression of both genes flanking the insertion site in mature rosette leaves examined by RT-PCR.
   Amplification using oligonucleotides specific for the actin gene were used for analyzing equal loading and unsaturation of the PCR reaction. Wt, wild type; *ggl* is the activation tag mutant.

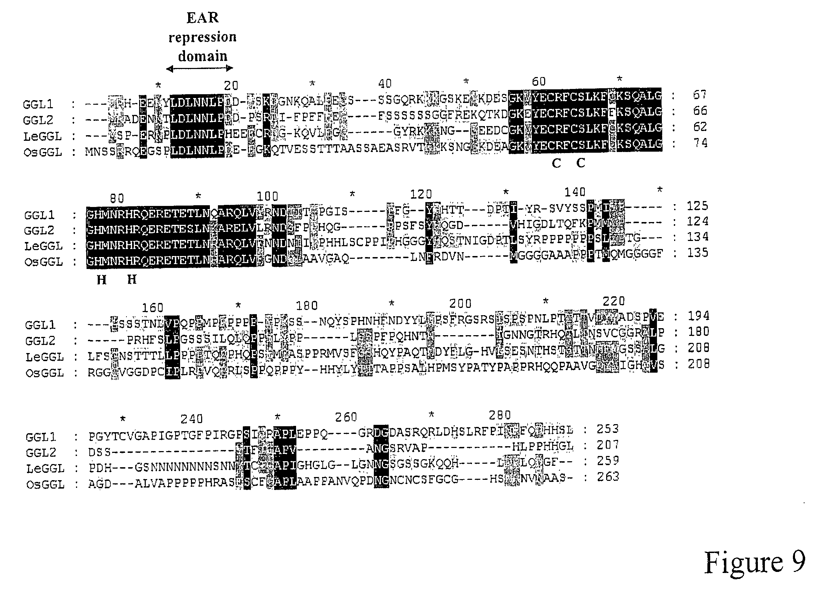

Figure 9: Protein alignment of the *GGL* genes and their putative orthologues from tomato *(LeGGL)* and rice *(OsGGL).*
   Black, gray and light gray indicate 100%, 75% and 50% identify, respectively. C and H are the cysteine and histidine residues part of the C2H2 single zinc-finger domain present in these proteins.

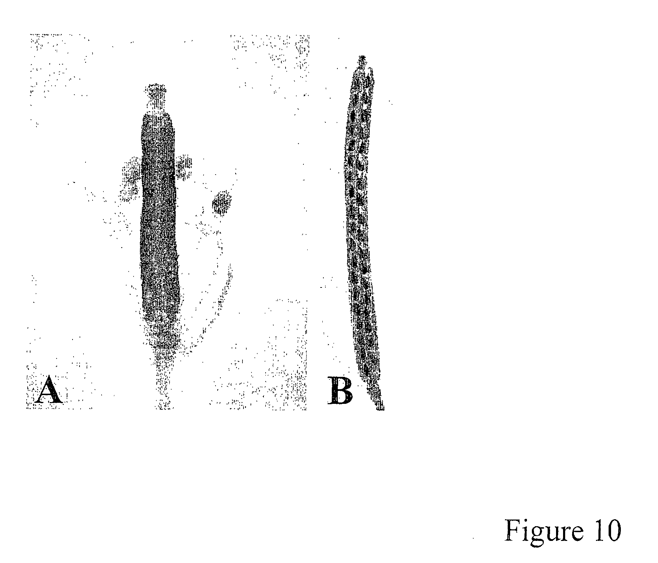

Figure 10: *GGL1* expression in developing fruit.
   (A) GUS expression was detected in stage 15 of flower development in the valves but not in the replum (Ferrandiz et al., 1999).
   (B) GUS expression could not be detected in mature silique (stage 17).

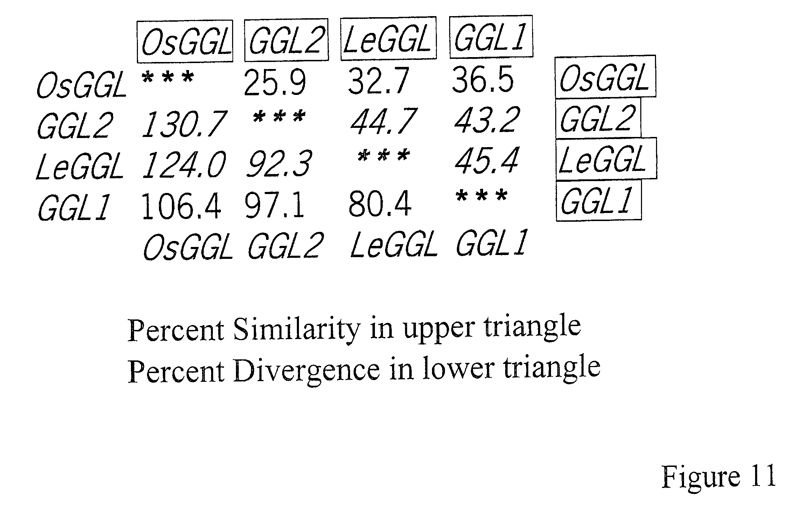

Figure 11: Similarity between the GGL proteins from Arabidopsis (GGL1, GGL2), rice (OsGGL) and tomato (LeGGL).

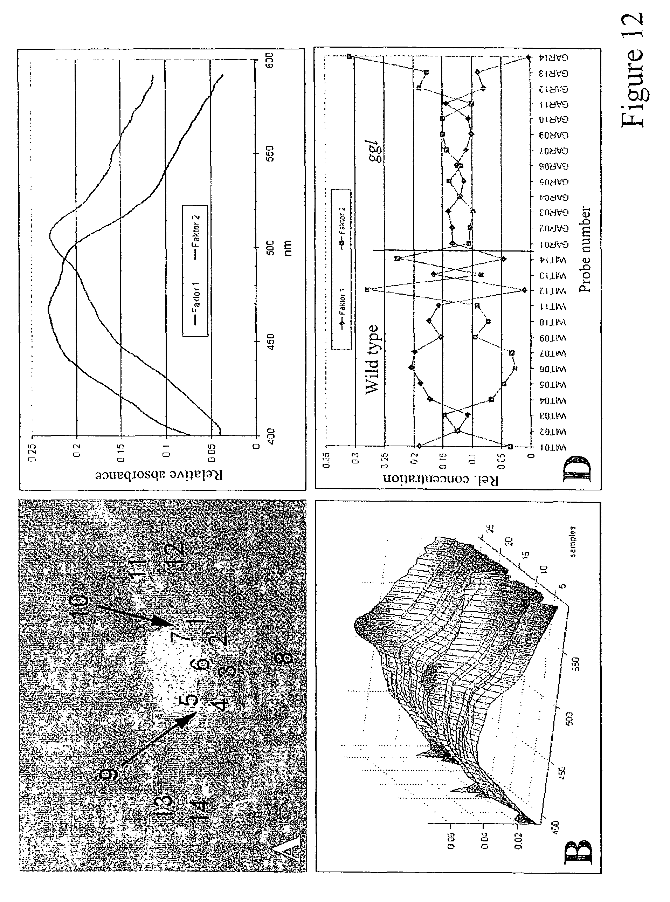

Figure 12: Composition change in lignin due to GGL overexpression and mis-expression in mature siliques.
   (A) Auto fluorescence of a Wild type cross section. Numbers indicate the positions were micro spectral photometric measurements were recorded.
   (B) Auto fluorescence spectra of Wild type (1 to 13) and *ggl* (14-26, location 8 not shown), excitation at 360 nm.
   (C) Loading plot of factor 1 and factor 2 as a result of MCR of auto fluorescence spectra.
   (D) Score plot representing the contribution (weighting) of the two factors in each spectrum (470 nm and 515 nm for factor 1 and factor 2, respectively) and showing the results for each location measured from a cross section of Wild type and *ggl* siliques.
Figure 13: (A) Nucleotide sequence of the *Arabidopsis thaliana* GGL1 gene. Open reading frame is indicated by bold startcodon ATG and stopcodon TGA. (B) Amino acid sequence of the GGL1 protein. Note the EAR motif LDLNNLP at amino acid position 8 and the zinc finger motif KSQALGG at amino acid position 62.

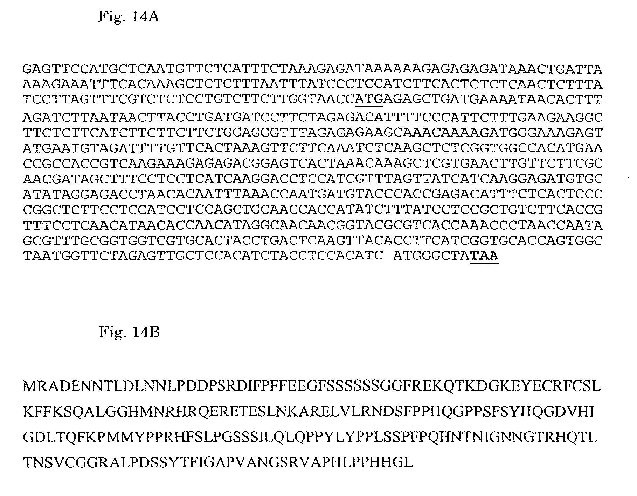

Figure 14: (A) Nucleotide sequence of the *Arabidopsis thaliana* GGL2 gene. Open reading frame is indicated by bold startcodon ATG and stopcodon TAA. (B) Amino acid sequence of the GGL2 protein. Note the EAR motif LDLNNLP at amino acid position 9 and the zinc finger motif KSQALGG at amino acid position 61.

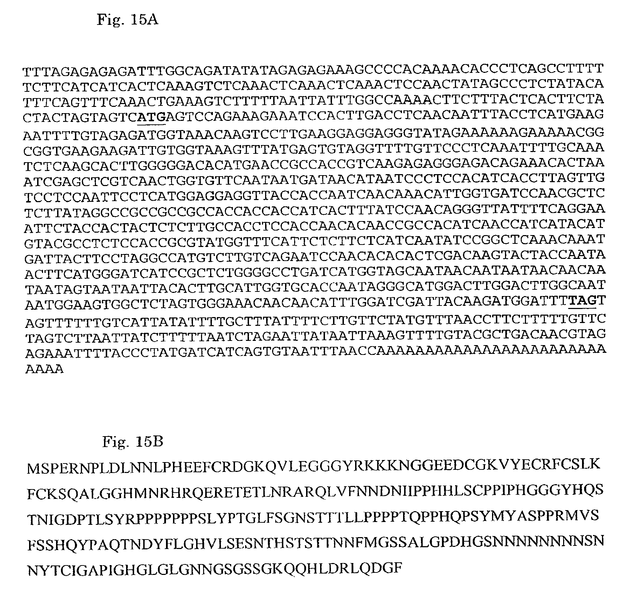

Figure 15: (A) Nucleotide sequence of the *Lycopersicon esculentum* LeGGL gene. Open reading frame is indicated by bold startcodon ATG and stopcodon TAG.
   (B) Amino acid sequence of the LeGGL1 protein. Note the EAR motif LDLNNLP at amino acid position 8 and the zinc finger motif KSQALGG at amino acid position 57.

### Definitions

"Recombinant DNA technology" refers to procedures used to join together DNA sequences as described, for example, in Sambrook et al., 1989, Cold Spring Harbor, NY: Cold Spring Harbor Laboratory Press.

A "plant" refers to any plant, particularly to plants of the family of *Brassicaceae*, more in particular *Brassica napus..*

"Plant cell" refers to the structural and physiological unit of the plant, comprising a protoplast and a cell wall. The plant cell may be in form of an isolated single cell or a cultured cell, or as a part of higher organized unit such as, for example, a plant tissue, or a plant organ.

"Plant material" refers to leaves, stems, roots, seeds, flowers or flower parts, fruits, pollen, pollen tubes, ovules, embryo sacs, egg cells, zygotes, embryos, seeds, cuttings, cell or tissue cultures, or any other part or product of a plant.

"Expression" refers to the transcription and/or translation of an endogenous gene or a transgene in plants. In the case of antisense constructs, for example, expression may refer to the transcription of the antisense DNA only.

"Polynucleotide construct" as used herein means a nucleotide sequence capable of directing expression of a particular nucleotide sequence in an appropriate host cell, comprising a promoter operably linked to the nucleotide sequence of interest which is optionally operably linked to 3' sequences, such as 3' regulatory sequences or termination signals. It also typically comprises sequences required for proper translation of the nucleotide sequence. The coding region usually codes for a protein of interest but may also code for a functional RNA of interest, for example antisense RNA or a nontranslated RNA that, in the sense or antisense direction or as a doublestranded sequence, inhibits expression of a particular gene, e. g. through antisense inhibition or RNA interference (RNAi). The polynucleotide construct comprising the nucleotide sequence of interest may be chimeric, meaning that the nucleotide sequence is comprised of more than one DNA sequences of distinct origin which are fused together by recombinant DNA techniques resulting in a nucleotide sequence which does not occur naturally, and which particularly does not occur in the plant to be transformed. The polynucleotide construct may also be one which is naturally occurring but has been obtained in a recombinant form useful for heterologous expression. Typically, however, the polynucleotide construct is heterologous with respect to the host, i.e., the particular DNA sequence of the polynucleotide construct does not occur naturally in the host cell and must have been introduced into the host cell or an ancestor of the host cell by a transformation event. The expression of the nucleotide sequence in the polynucleotide construct may be under the control of a constitutive or tissue-specific promoter or of an inducible promoter which initiates transcription only when the host cell is exposed to some particular external stimulus. In the case of a multicellular organism, such as a plant, the promoter can also be specific to a particular tissue or organ or stage of development. A nuclear polynucleotide construct is usually inserted into the nuclear genome of a plant and is capable of directing the expression of a particular nucleotide sequence from the nuclear genome of said plant.

"Gene" refers to a coding sequence and associated regulatory sequences wherein the coding sequence is transcribed into RNA such as mRNA, rRNA, tRNA, snRNA, sense RNA or antisense RNA. Examples of regulatory sequences are promoter sequences, 5' and 3' untranslated sequences and termination sequences. Further elements that may be present are, for example, introns.

"Heterologous" as used herein means of different natural or of synthetic origin. For example, if a host cell is transformed with a nucleic acid sequence that does not occur in the untransformed host cell, that nucleic acid sequence is said to be heterologous with respect to the host cell. The transforming nucleic acid may comprise a heterologous promoter, heterologous coding sequence, or heterologous termination sequence.

Alternatively, the nucleic acid which is introduced into a plant (cell) may be completely heterologous or may comprise any possible combination of heterologous and endogenous nucleic acid sequences. Similarly, heterologous refers to a nucleotide sequence derived from and inserted into the same natural, original cell type, but which is present in a non-natural state, e. g. a different copy number, or under the control of different regulatory elements.

"Marker Gene" a gene encoding a selectable or screenable trait.

A regulatory DNA sequence is said to be "operably linked to" or "associated with" a DNA sequence that codes for an RNA or a protein if the two sequences are situated such that the regulatory DNA sequence affects expression of the coding DNA sequence.

"Regulatory elements" refer to sequences involved in conferring the expression of a nucleotide sequence. Regulator elements comprise a promoter operably linked to the nucleotide sequence of interest and, optionally, 3' sequences, such as 3' regulatory sequences or termination signals. They also typically encompass sequences required for proper translation of the nucleotide sequence.

A "promoter" refers to a DNA sequence that initiates transcription of an associated DNA sequence. The promoter region may also include elements that act as regulators of gene expression such as activators, enhancers, repressors, binding sites for DNA binding proteins, and/or sequences that convey tissue specificity.

"Overexpression" or "Overproduction" refers to a level of expression or production of a protein or metabolite which exceeds the demand and may lead to accumulation and storage.

A "screenable marker gene" refers to a gene whose expression does not confer a selective advantage to a transformed cell, but whose expression makes the transformed cell phenotypically distinct from untransformed cells.

A "selectable marker gene" refers to a gene whose expression in a plant cell gives the cell a selective advantage. The selective advantage possessed by the cells transformed with the selectable marker gene may be due to their ability to grow in the presence of a negative selective agent, such as an antibiotic or a herbicide, compared to the growth of non-transformed cells. The selective advantage possessed by the transformed cells, compared to non-transformed cells, may also be due to their enhanced or novel capacity to utilize an added compound as a nutrient, growth factor or energy source. Selectable marker gene also refers to a gene or a combination of genes whose expression in a plant cell gives the cell both, a negative and a positive selective advantage.

"Transformation" refers to introduction of a nucleic acid into a cell and in particular, the stable integration of a DNA molecule into the genome of an organism of interest.

### Detailed Description of the Invention

We now have found a new mutant line in *Arabidopsis* in which there is a significant reduction in pod shattering due to an alteration of the lignification in the silique. A new pattern of lignification, not observed in hitherto known mutants, as discussed above, was detected, in which lignified valve margin cells from both sides of the replum were interconnected by lignified cell layers. The formation of such a "lignification bridge" will result in the complete encircling of the silique with lignified cells, which is the cause for the reduction in pod-shattering.

This mutant was discovered by activation gene tagging (as discussed in the Examples) and was named *GARGOYLE (GGL)* because the overall phenotype of the plant that was characterized by long and downwards curled rosette leaves, lack of a petiole, curly, twisted and often red-colored stems, and the presence of often serrated cauline leaves (or bracts) at the bottom of the silique.

It appeared that the activation tag was inserted near gene At1g68480 . This gene codes for a protein, of which the sequence is given in Fig. 13B, which contains a single zinc finger domain of the Cys₂/His₂ (C2H2) type, and a putative repression domain in its N-terminal region, most similar to the so-called EAR (ERF-associated Amphiphilic Repression) motif identified in other transcription factors in plants.

The zinc finger contains an QALGGH motif, which places it in the group of the EPF type zinc fingers, which have a basic structure of antiparallel, two-stranded β-sheets and a short α-helix. The zinc finger domain is an unusually small (25 to 30 aa residues), self-folding domain in which Zn is a critical component of its tertiary structure, and which is able to bind nucleic acid. Each motif binds 1 atom of Zn in a tetrahedral array to yield a finger-like projection, which interacts with nucleotides in the major groove of the nucleic acid (with the ability to bind both DNA and RNA). In the C2H2 class the first pair of zinc coordinating residues are cysteines, while the second pair are histidines.

The EAR motif is a conserved motif originally identified in Class II ERF (ethylene responsive element-binding factor) transcriptional repressors (Ohta et al, 2001, Plant Cell 13: 1959-1968). The EAR motif (L/FDLNL/FxP) has been identified up to date in the C-terminal region of transcriptional repressor proteins. An additional gene, which is a close homologue of *GGL* was also identified in the *Arabidopsis* genome (termed *GGL2,* Fig. 14, At1g13400). Figure 9 shows an amino acid sequence alignment of the predicted GGL proteins (GGL1 and GGL2). GGL-homologues were also found in tomato and rice. Another protein that also has an EAR-like motif in its C-terminal region is *SUPERMAN* which is an active repressor involved in flower development. Ectopic expression of the *SUPERMAN* gene that lacked the repression domain resulted in a phenotype similar to that of *superman* mutants, with flowers having extra stamens (Hiratsu *et al.,* 2002, FEBS Lett 514: 351-354). Figure 9 shows the alignment of the GGL1 protein with its homologue GGL2 from *Arabidopsis* and the putative orthologue proteins from rice and tomato. All four proteins share between 30 to 38% identity in between each other (Figure 11).

It is the hypothesis that the GGL protein works together with the RPL protein (encoded by the gene At5g02030) obtained through the *replumless* or *rpl* mutant (Roeder, A. et al, Current Biology, p. S1-S4, 2003) as an agonistic/antagonistic couple, where the GGL protein possibly acts as a repressor molecule for the RPL gene or protein. Overexpression of GGL thus inactivates RPL expression and the effects of overexpression of GGL resemble the effects obtained by (specific) knock-out of RPL expression (Roeder, A.H.K., et al., supra). This also indicates that mutant forms of GGL and/or RPL which are different from their original counterparts but for the recognition and/or binding to RPL and/or GGL, respectively, would be able to give the same phenotype if the overall effect of these mutant forms would be blocking the effects that RPL normally has.

Next to the increased lignification in the siliques the above described *ggl* mutant (like has been described for RPL knock-outs) also showed ectopic lignification in the stems. Sectioning of the inflorescence stems revealed increased interfascicular (between the vascular bundles) deposition of lignin.

One embodiment of the invention now is to use the sequences of the invention to transform plants for reduction of pod shattering. However, it is preferred to limit expression of the protein encoded by the sequences of the invention to avoid economically disadvantageous aberrant phenotypes. One way to accomplish this is to limit expression to the silique, more specifically to the replum, by using replum-specific promoters. All promoters which would normally specifically show expression in the replum would be useful in this respect. Examples of the promoters that can be used are the CYCD3;2 promoter (Swaminathan et al, 2000 Plant Physiol, 124: 1658); the ABF3/ABF4 promoters Kang et al 2002, Plant Cell 14: 343-357); and the RPL promoter (Roeder et al 2003 supra) and other such promoters can be found by the person skilled in the art.

Alternatively, inducible or gene switch promoters can be used, which need not to be tissue specific, as long as they show expression in the replum. The advantage of such inducible promoters is that they are silent when not induced, and thus the plants can develop and produce fruits unhindered by the transgenic construct. Induction then is necessary at a time which allows the "lignification bridge" to be formed, thereby preventing early pod shattering. Inducible promoters include any promoter capable of increasing the amount of gene product produced by a given gene in response to exposure to an inducer. In the absence of an inducer, the DNA sequence will not be transcribed. Typically, the factor that binds specifically to an inducible promoter to activate transcription is present in an inactive form which is then directly or indirectly converted to the active form by the inducer. The inducer may be a chemical agent such as a protein, metabolite (sugar, alcohol, *etc*.), a growth regulator, herbicide, or a phenolic compound or a physiological stress imposed directly by heat, salt, wounding, toxic elements *etc*., or indirectly through the action of a pathogen or disease agent such as a virus. A plant cell containing an inducible promoter may be exposed to an inducer by externally applying the inducer to the cell such as by spraying, watering, heating, or similar methods. Inducible promoters are known to those familiar with the art and several exist that could conceivably be used to drive expression of the *GGL* gene. Inducible promoters suitable for use in accordance with the present invention include, but are not limited to, the heat shock promoter, promoters inducible by the mammalian steroid receptor system and any chemically inducible promoter. Examples of inducible promoters include the inducible 70 kD heat shock promoter of *Drosophila melanogaster* (Wing et al 1989, Mol Gen Genet, 219: 9-16) and the alcohol dehydrogenase promoter which is induced by ethanol (Freeling, M. *et al.,* Ann. Rev. Genet. 19, 297-323; Nagao, R.T. *et al.,* in: Miflin, B.J. (ed.) Oxford Surveys of Plant Molecular and Cell Biology, Vol. 3., pp. 384-438, Oxford Univ. Press, 1986). A promoter that is inducible by a simple chemical is particularly useful. Such simple or common chemicals are used in the induction of so-called gene switch promoters. Examples of gene switch promoters include the alcA/alcR gene switch promoter as described in published International Patent Application No. WO 93/21334; the GST promoter, as described in published International Patent Application Nos. WO 90/08826 and WO 93/031294; and the ecdysone switch system as described in published International Patent Application No. WO 96/37609. In such switch systems, the timing of gene expression is controlled by application of an external chemical. The switch chemical may be applied as a spray or vapor to all or part of the transgenic plant or as a root drench. Examples of suitable switch chemicals are provided in the above references describing switch promoter systems. The external chemical stimulus is preferably an agriculturally acceptable chemical, the use of which is compatible with agricultural practice and is not detrimental to plants or mammals. Inducible switch promoter systems preferably include one or two component systems; nevertheless, systems comprising more than two components are encompassed by the present invention. The switch system may be driven by a constitutive promoter or by a tissue or organ-specific promoter, whereby the target gene is only switched on in a target tissue or organ. The alcA/alcR switch promoter system is particularly preferred. In the alcA/alcR promoter switch system, the preferred chemical inducer is ethanol, in either liquid or vapour form. One of the main advantages of the use of ethanol is that small quantities of ethanol generate high levels of expression. The alcA/alcR inducible promoter system is a two-component system involving DNA sequences coding for the alcA promoter and the alcR protein, the expression of which is placed under the control of desired promoters. The alcR protein activates the alcA promoter in the presence of an inducer and any gene under the control of the alcA promoter (in this case the *GGL* gene), will therefore be expressed only in the presence of that inducer. With such a system the activity of the gene construct can be limited both by place and by time. Other gene-switch systems and/or inducible promoters are known in the art and would also be equally applicable.

A similar approach can be taken when the overexpression of *GGL* is used to increase lignification in the stem. It is then preferable to switch on expression of the gene after the stem has been developed, to be able to only increase the thickness of the stem and not to interfere with the growth of the stem. For this a stem expressible promoter such as the potato ST-LS1 promoter which is stem and leaf specific (Stockhaus et al. 1987, Nucleic Acids Res.15(8):3479-91); a xylem and interfasicular promoter such as the C4H (Franke et al 2000 Plant J 22: 223-234) or a switchable constitutive promoter such as the CaMv 35S promoter can be used.

The recombinant DNA constructs for use in the methods according to the present invention may be constructed using recombinant DNA technology well known to persons skilled in the art.

Polynucleotide constructs for expression of a gene in the plant nucleus preferably comprise appropriate 3' sequences, such as 3' regulatory sequences or transcription terminators, to be operably linked downstream of the heterologous nucleotide sequence. Several such terminators are available and known in the art (e. g. tm1 from CaMV, PotPI II from potato, E9 from rbcS). Any available terminator known to function in plants can be used in the context of this invention. Numerous other sequences can be incorporated into polynucleotide constructs for expression of a DNA molecule described in this invention. These include sequences which have been shown to enhance expression such as intron sequences (e. g. from Adhl and bronzel) and viral leader sequences (e. g. from TMV, MCMV and AMV).

The polynucleotide construct comprises a recombinant polynucleotide for overexpression of the *GGL*-gene. Said gene preferably comprises a nucleic acid which codes for a protein according to Fig. 13B or a functional fragment thereof. A functional fragment of said protein is defined as a protein which is highly homologous to the protein depicted in Fig. 13B and which remains functional when expressed in a plant, wherein said functionality means that it is able to induce lignification of green parts of the plant, preferably the stem, or to induce formation of the lignification bridge in the replum of a silique.

Highly homologous in this sense means that an amino acid sequence has a sequence homology of more than 50%, preferably more than 70%, more preferably more than 80% and most preferably more than 90% with the above mentioned sequence.

For calculation of percentage identity (or homology) the BLAST algorithm can be used (Altschul et al., 1997 Nucl. Acids Res. 25:3389-3402) using default parameters or, alternatively, the GAP algorithm (Needleman and Wunsch, 1970 J. Mol. Biol. 48:443-453), using default parameters, which both are included in the Wisconsin Genetics Software Package, Genetics Computer Group (GCG), 575 Science, Madison, Wisconsin, USA. BLAST searches assume that proteins can be modeled as random sequences. However, many real proteins comprise regions of nonrandom sequences which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Wooten and Federhen, 1993 Comput. Chem. 17:149-163) and XNU (Claverie and States, 1993 Comput. Chem. 17:191-201) low-complexity filters can be employed alone or in combination.

As used herein, 'sequence identity' or 'identity' or 'homology' in the context of two protein sequences (or nucleotide sequences) includes reference to the residues in the two sequences which are the same when aligned for maximum correspondence over a specified comparison window. When percentage of sequence identity is used in reference to proteins it is recognised that residue positions which are not identical often differ by conservative amino acid substitutions, where amino acids are substituted for other amino acid residues with similar chemical properties (e.g. charge or hydrophobicity) and therefore do not change the functional properties of the molecule. Where sequences differ in conservative substitutions, the percentage sequence identity may be adjusted upwards to correct for the conservative nature of the substitutions. Sequences, which differ by such conservative substitutions are said to have 'sequence similarity' or 'similarity'. Means for making these adjustments are well known to persons skilled in the art. Typically this involves scoring a conservative substitution as a partial rather than a full mismatch, thereby increasing the percentage sequence identity. Thus, for example, where an identical amino acid is given a score of 1 and a non-conservative substitution is give a score of zero, a conservative substitution is given a score between 0 and 1. The scoring of conservative substitutions is calculated, e.g. according to the algorithm of Meyers and Miller (Computer Applic. Biol. Sci. 4:11-17, 1988).

Preferably the nucleic acid which codes for the GGL protein comprises the sequence as depicted in Fig. 13B.

The polynucleotide construct of the present invention is preferable constructed such that it comprises at least and in operable linkage a first promoter that is functional in plants, preferably a replum-specific promoter, a nucleotide sequence encoding a GGL protein, and a terminator. Optionally the polynucleotide may comprise a gene sequence encoding a selectable or screenable trait operably linked to regulatory sequences for expression.

The recombinant gene constructs may be inserted into a vector, which may be commercially available, suitable for transforming into plants and suitable for expression of the gene product in the transformed cells. Preferably used are binary vectors which are useful for plant transformation using *Agrobacterium.*

Transformation of plants, in particular transformation of oilseed rape and other commercially important *Brassicaceae* has been practiced since the beginning of plant transformation technique development. In principle any transformation method may be used to introduce chimeric DNA according to the invention into a suitable ancestor cell. Methods may suitably be selected from the calcium/polyethylene glycol method for protoplasts, electroporation of protoplasts, microinjection into plant material, (DNA or RNA-coated) particle bombardment of various plant material, infection with (non-integrative) viruses, in planta *Agrobacterium tumefaciens* mediated gene transfer by infiltration of adult plants or transformation of mature pollen or microspores (EP 0 301 316) and the like. A preferred method according to the invention comprises *Agrobacterium-*mediated DNA transfer. Especially preferred is the use of the so-called binary vector technology as disclosed in EP 0 120 516 and U.S. Patent 4,940,838.

A method for production of a transgenic plant or plant part according to the invention may comprise the step of selecting transformed plants or plant parts. Generally after transformation, plant cells or cell groupings are selected for the transfer with the polynucleotide construct comprising the DNA-sequence with the genes encoding the various enzymes or blocking mechanisms according to the invention, following by steps know to the skilled person by which the transformed material is regenerated into a whole plant and evaluating the transformed plant for the overproduction of glycerol.

Selectable markers, which may be included as a part of the introduced recombinant DNA, are used to select transformed cells (those containing recombinant DNA) over untransformed cells. Examples of suitable markers include genes that provide antibiotic or herbicide resistance. Cells containing the recombinant DNA are capable of surviving in the presence of antibiotic or herbicide concentrations that kill untransformed cells. Examples of selectable marker genes include the bar gene which provides resistance to the herbicide Basta; the nptII gene which confers kanamycin resistance; the hpt gene which confers hygromycin resistance; and the cah gene which gives resistance to cyanamid. An entire plant can be generated from a single transformed plant cell through cell culturing techniques known to those skilled in the art.

A process for obtaining a transgenic GGL overproducing plant according to the invention may in an alternative embodiment comprise introducing a vector according to the invention into an ancestor plant, and then producing said transgenic GGL overproducing plant from said ancestor plant.

Yet another alternative embodiment for obtaining a transgenic GGL overproducing plant according to the invention may comprise introducing a polynucleotide construct according to the invention into a suitable vector for transforming a plant part to produce a transformed plant part, and then regenerating said transgenic GGL overproducing plant from said transformed plant part.

Following DNA transfer and regeneration, putatively transformed plants may be evaluated, for instance using Southern analysis, for the presence of the recombinant DNA according to the invention, copy number and/or genomic organization. In addition, or alternatively, expression levels of the newly introduced DNA may be undertaken, using Northern and/or Western analysis, techniques well known to persons having ordinary skill in the art.

The following enabling Examples serve to further illustrate the invention, and are not intended to define limitations or restrict the scope of the subject invention.

### EXAMPLES

### Example 1 - Material and Methods

### 1.1 Plant Material

All plants, including the activation tag population (Marsch-Martinez *et al.,* 2002, Plant Physiol. 129: 1544-1556) and transgenic lines were grown in the greenhouse at around 22°C. The *shp1*/*shp2* double mutant was in the Columbia ecotype and *ful-1* in the Ler background.

### 1.2 Isolation of Flanking DNA and Sequence Analysis

DNA was isolated according to Pereira and Aarts (1998, Transposon tagging with the En-I system, Totowa, NJ, Humana Press), from two leaves or young flower buds, and 10 ng of genomic DNA was used for Thermal Asymmetric Interlaced-PCR (TAIL PCR) as described by (Marsch-Martinez *et al.,* 2002, supra). A re-PCR was generally performed before sequencing the amplified fragments, and identifying the insert position in the Arabidopsis genome using a BlastN algorithm (Altschul *et al.* 1990, J. Mol. Biol. 215:403-410). Multiple sequence alignments were performed using CLUSTAL X (Thompson *et al*. 1997, Nucl. Acid Res. 25, 4876-4882) and DNASTAR (DNASTAR Inc. Madison, WI) while the GENEDOC (Nicholas *et al.* 1997, EMBNET News 4, 1-4) and TreeView (Page, 1996, Comp. Applic. Biosci. 12: 357-358) programs were used for editing the alignment and producing the phylogenetic tree, respectively. Phylogenetic analysis including bootstrapping was conducted as described by Lucker *et al.* (2002, Eur. J. Biochem. 269, 3160-3171).

### 1.3 Generation of Plant Transformation Constructs and Transgenic Arabidopsis

Fragments encompassing the full length coding regions were amplified (using pfu DNA polymerase) from genomic DNA (At1g68480 for *GGL1,* and At1g13400 for *GGL2)* to generate the three overexpression constructs. The cDNA (produced as described below in Gene Expression Analysis) and genomic DNA used for amplification were from the Arabidopsis ecotype Columbia. Oligonucleotides AP55 and AP44 were used to amplify *GGL1,* while oligonucleotides AP57 and AP58 were used to amplify *GGL2.*

Both pairs of oligonucleotides introduced BamHI and SstI restriction sites to the amplified fragments at their 5' and 3', respectively, which were utilized to ligate the coding region fragments to the BamHI and SstI sites in the pBI121 binary vector (Clontech, Palo Alto, CA) in between a 35S promoter of the cauliflower mosaic virus (CaMV) and a nopaline synthase (NOS) terminator. The BamHI and SstI fragment of the *GGL1* gene was utilised to generate a third construct in which the it was placed under the control of its native promoter region (Xbal and BamHI fragment of its 1.6kb upstream region) and coupled to the 35S CaMV enhancer tertramer (used to generate the enhancer line).

For generating the promoter::GUS constructs, fragments upstream to the ATG codon of the *GGL1* gene (either 1.6kb without the first exon and intron or 1.7kb including the first exon and intron) were amplified from genomic DNA (ecotype Columbia) using Taq DNA polynerase and oligonucleotides which introduced HindIII NcoI restriction sites at the 5' and 3', respectively. This allowed ligation of the fragments to the XbaI and NcoI sites in a modified pBinPlus vector (van Engelen *et al,* 1995 Transgenic Research 4: 288-290) upstream of the β-glucuronidase (GUS) reporter gene. The oligonucleotides AP53 and AP54 were used to amplify the *GGL1* 1.6kb upstream region and AP53 and AP67 for the *GGL1* 1.7kb upstream region. In all cases fragments were A- tailed and introduced to the pGEM-T Easy vector as described by the manufacturer (Promega) and subsequently sequenced from both sides before digestion and ligation to the Binary vector. PCR, restriction digests, plasmid DNA isolation and gel electrophoresis were performed using standard protocols. The constructs were introduced into the plants using the floral dipping transformation method (Clough and Bent, 1998, Plant J. 16, 735-743). The seeds were plated on one-half-strength Murashige and Skoog medium (1/2MS; Murashige and Skoog, 1962, Physiol. Plant. 15, 473-497) and seedlings selected on 50 mg/L kanamycin were subsequently transferred to the greenhouse.

### Oligonucleotides:

AP55 (5'- CGGATCCTTCCCACTATGAGGCATGAG –3')

AP44 (5'- CGAGCTCTCAGAGCGAGTGATGATCTT- 3')

AP57 (5'- CGGATCCAATGACGGAGGTACGTTG –3')

AP58 (5'- CGAGCTCTTATAGCCCATGATGTGGAGG –3')

AP53 (5'- CAAGCTTGCTTCCGTGAGAGTGAATTGTG –3')

AP54 (5'- CCCATGGGTGGAAATGAGAGATTGGCG- 3')

AP67 (5'- CCCATGGGTAATTCTCCTCATGCCTCCTGC –3')

### 1.4 Gene Expression Analyses

Total RNA for Reverse Transcriptase-PCR (RT-PCR) was isolated from mature, green, rosette leaves derived from 4 weeks old shn activation tag mutant and wild type (ecotype WS) plants using the TrizolReagent as described by the manufacturer (Invitrogen, Life technologies). Approximately 1 µg of total RNA was used for DNase I treatment and cDNA synthesis (using SuperScriptII reverse transcriptase) as described by the supplier (Invitrogen, Carlsbad, CA). The cDNA was diluted 50 times and used for amplification using specific oligonucleotides for the actin gene to equalise the concentrations of the cDNA samples.

### Actin Primers:

RACTP1, 5'- GCGGTTTTCCCCAGTGTTGTTG -3'

RACTP2, 5'- TGCCTGGACCTGCTTCATCATACT -3'

Subsequently the diluted cDNA was utilized to perform a PCR reaction using specific oligonucleotides designed to amplify the two genes flanking the insertion site. Oligonucleotides AP43 and AP44, were used to amplify the At1g68480 gene *(GGL1)* and AP45 and AP46, to amplify At1g68470. The reaction conditions for PCR included a denaturing step of 95°C for 3 min, followed by 35 cycles of 1 min at 95°C, 1 min at 55°C, and 1.5 min at 72°C, ending with an elongation step of 5 min at 72°C. For the control PCR with actin oligonucleotides, 30 amplification cycles were used.

AP43 5'- CGGATCCATGATATATCCTGGAAGCTC -3'

AP44 5'- CGAGCTCTCAGAGCGAGTGATGATCTTG-3'

AP45 5'- CGGATCCCCATGACTTTCAATAAGAGGC -3'

AP46, 5'- CGTCGACGAGTAGCACAAAACTATAGGTC -3'

### 1.5 GUS Staining and Microscopy

Tissues from various organs either from soil grown plants or seedlings grown on 1/2 MS in vitro were analyzed for their GUS expression patterns. The GUS solution contained 100Mm sodium phosphate buffer, pH 7.0, 0.5 mg/ml 5-bromo-4-chloro-3-indolyl β-D glucoronic axid (X-Gluc, Duchefa, The Netherlands), 0.1% Triton, and 0.5 mM each of potassium ferri/ferrocyanide. Samples were vacuum infiltrated and incubated at 37°C for 16 to 24 h and depleted from chlorophyll in 70% ethanol. Observation were conducted either under the binocular (WILD M3Z of Heerbrugg Switzerland, type-S), or with a light microscope (Zeiss) and an RS Photometries CoolSNAP camera (MediaCybernetics®) was used to take the digital images, with the corresponding CoolSNAP software.

For Scanning Electron Microscopy (SEM) after overnight fixation in FAA (5% glacial acetic acid; 3.7% formaldehyde; 50% ethanol) fixation, dehydration was conducted in a series of acetone 10-30-50-70-90-100 3x. Then in series mixture of 100% acetone (A) and 100% chloroform (C) 3:1--1:1--1:3 then 100% chloroform. And then back to 100% acetone in the same series backwards. Samples were left in 100% acetone until critical point drying.

Samples were glued on a sample holder with conductive carbon cement (Leit- C, Neubauer Chemikalien, Germany) and subsequently frozen in liquid nitrogen. The samples were transferred under vacuum to a dedicated cryo-preparation chamber (Oxford cryo-system, CT 1500 HF, Eynsham, UK) onto a sample stage at -90° C. Cryo-fractures were made at approximately - 150°C using a cold (-196°C) scalpel blade. The fractured samples were freeze dried for 3 min at -90°C in vacuum (3x10-7 Pa) to remove water vapor contamination. After the sample surface was sputter-coated with 10 nm Platinum it was transferred to the cold sample stage (-190°C) inside the Cryo-FESEM (JEOL 6300F Field Emission SEM, Japan, Tokyo) and subsequently analyzed with an accelerating voltage of 5 kV. Images were digitally recorded (Orion, Belgium).

### Example 2 - Identification of the ggl Mutant

We screened an *Arabidopsis* activation tag population (Marsch-Martinez et al, supra) for lines altered in plant architecture and identified one such mutant which we named *gargoyle (ggl).* Progeny analysis of the self-pollinated *ggl* mutant line suggested a dominant mutation (three quarters of the plants exhibited the *ggl* phenotype).

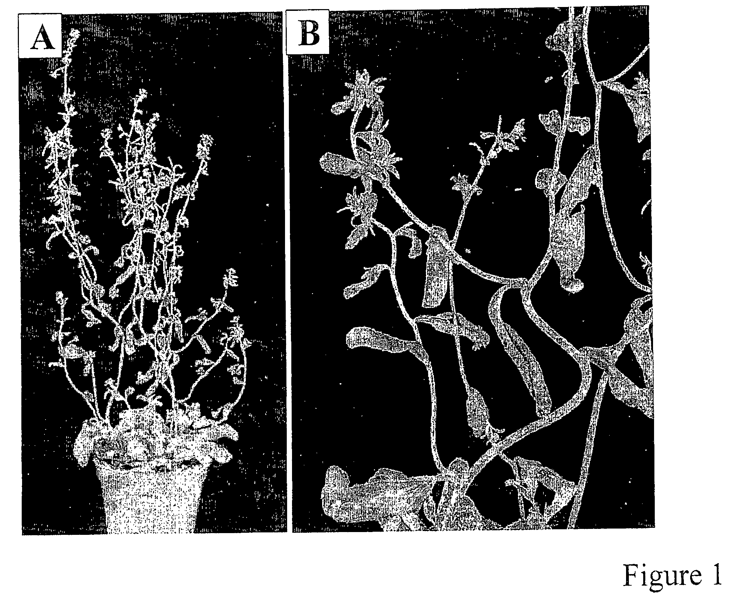

The *ggl* mutant line showed an array of phenotypes, which dramatically altered the plant architecture (Figure 1). Rosette leaves of *ggl* plants were longer then wild-type leaves, showed epinasty and in most cases lacked petioles (forming a drop-like shape), while cauline leaves were often longer and twisted. The overall inflorescence structure was unusual as flower pedicels were on average 15% the length of wild type pedicels (average pedicel length in wild type was 11.4 mm whereas *ggl* pedicels were on average 1.8 mm long, n = 70 per genotype). The flowers were subtended by a bract which differed in size and shape and was often serrated. Flowers, mainly petals were bigger in size but fertility was normal. Siliques were slightly shorter in the *ggl* mutant plants and often were crescent-shaped. The orientation of flowers and siliques was altered *in ggl* plants, as on average 54% of the pedicels had an angle between 90° to 180° (pedicels bend downwards). In wild type plants, the pedicel angle was in all cases bellow 90", pointing upwards (9 plants examined per genotype; n = 132 and 247 for *ggl* and wild type plants, respectively).

Phylotaxy was strongly affected since both the regular spiral arrangement of flowers and their distance along the stem on wild type stems was disrupted on *ggl* stems. The phylotactic arrangement of side shoots was also affected and they seemed to be randomly formed along the main flowering stem. Plants showing a strong phenotype occasionally produced side shoots which contained only leaves without flowers. Another, dramatic phenotype was the fusion of rosette or cauline leaves at their proximal part to stems of side shoots. Stems of side shoots often showed ectopic blade tissue outgrowth on their proximal part. The *ggl* mutant plants showed delayed flowering and generated the flowering stem two weeks later compared to wild type. Finally, cotyledons of *ggl* were also effected since they showed epinasty.

### Example 3 - Alterations to Lignin Load in Stems of the ggl Mutant

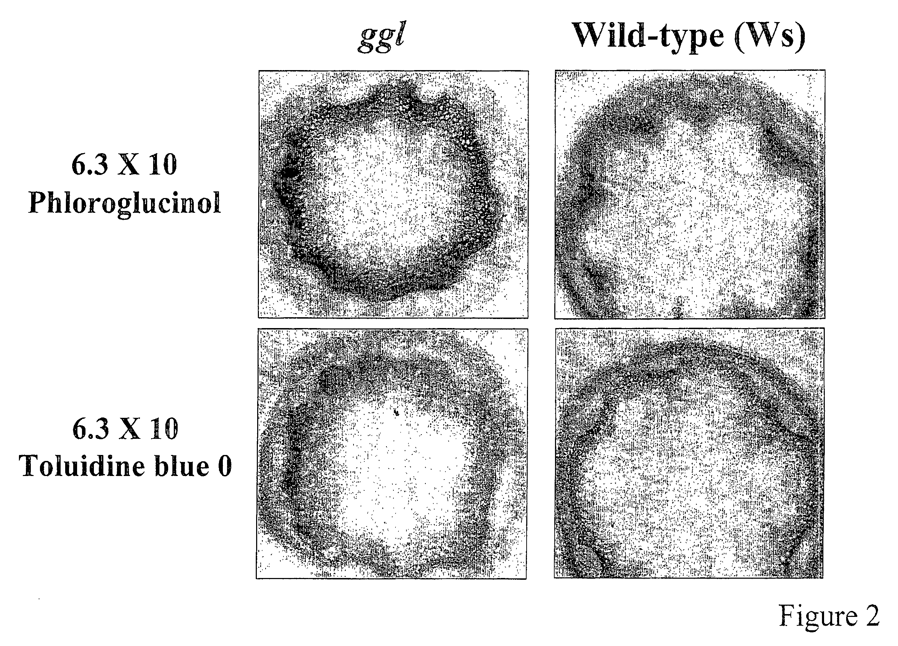

The flowering stems of *ggl* were inflexible and more brittle, often strongly twisted, exhibited bends at nodes, showed regions of reddening and could not stand as erect as wild-type. Stems of mature (six weeks old) flowering plants were divided into four equal segments (1-4), cross-sectioned, stained and examined by light microscopy. In wild type stems, starting from the second segment (below the apex), two to three layers of lignified interfascicular fibers are formed at the same level as the phloem, forming an arch-shaped pattern between the xylem (Figure 2). In the lowest segment (the more mature part) four to five layers of interfascicular fibers are formed. In the *ggl* mutant stem, most notably in the two bottom segments (3 and 4), the interfascicular fiber layer appeared thicker and with bigger lignified cells, was stretched to the level of the xylem and in some cases even penetrated to the central pith zone. As a result, the interfascicular fiber cells were not seen as arch-shaped (as in the wild-type) but rather formed a ring-like, heavily lignified structure. In addition, the mutant stem had wider vascular bundles and was not as round and symmetric when compared to wild type stems.

### Example 4 - Plants Overexpressing GGL1 do not Shatter

A striking characteristic of the *ggl* mutant phenotype was that mature pods showed a significant reduction in shattering. This was quantified in a population of *ggl* mutant plants: 20% show strong non-shattering that release seed only when twisted, 50% open only when clamped strongly between fingers, and 30% show wild-type like shattering that open when flicked.

*Arabidopsis* produces dry dehiscent fruit in which the two carpel valves are joined to the replum: a thin structure that separates the valves and is actually the outer margin of the septum. At the valve-replum boundary (valve margin), a narrow band of cells develops into the dehiscence zone. This is the region that eventually allows detachment of the valves from the replum, as its cells break down when the seeds are ready for dispersal (Liljegren *et al.,* 2000 supra). The actual reason for the fruits to open and release their seeds, is the tension that is created in the silique around the valve margins by localized lignification and cell wall hydrolysis in specialized areas (Liljegren *et al*., 2004 supra). Therefore, change in the level, spatial and temporal production of lignification may result in alteration to pod-shattering. Toluidine blue and phloroglucinol-HCL staining (the latter staining lignin) of mature siliques cross sections (stage 17, as defined by Smyth *et al.,* 1990) showed that lignified cells similar to the ones present normally in the valve margins of wild type siliques have been formed inside the replum region (Figure 3). The lignified cells connected the valve margin cells from both sides of the *ggl* mutant replum. The formation of such a "lignification bridge" resulted in the complete encircling of the silique with lignified cells, which is a likely explanation for the reduction in silique shattering. In some cases the "lignification bridge" was not completely closed as single or a few cells did not show lignification. This partial closer could occur only in one replum or in both in a single silique.

We used an *ADPG1* (At3g57510; endo-polygalacturonase) promoter-GUS line (Roberts *et al.,* 2000) as a molecular marker for valve margin cells in order to test its expression in the siliques of the *ggl* mutant. While wild type siliques express GUS specifically along the valve margin cells embracing the replum, while in *ggl* we detected ectopic expression ectopic GUS expression could be seen in the replum region (Figure 4). This suggests that valve margin-like cells have been formed in the *ggl* mutant.

To further examine the features of the cells formed in the *ggl* mutant replum we observed mature siliques using scanning electron microscopy (SEM). SEM analysis of wild type siliques illustrated the replum region which is limited in both sides by the narrow cells of the valve margins (Figure 5). In *ggl* mutant siliques the replum region is dramatically reduced and only a few layers of narrow cells resembling valve margin cells could be identified (Figure 5). The decrease in size of the replum in *ggl* was variable between siliques and in severe cases, valves were most close to each other.

In recent years several genes encoding proteins which regulate development of the valves, valve margins and the replum in the Arabidopsis carpel have been identified and characterized, mainly through the work of Yanofsky and co-workers (Liljegren *et al,* 2000 supra; Ferrándiz *et al* 2000 supra). A model for the regulatory network specifying valve margin development suggested that the FRUITFUL (FUL) protein, normally expressed in valves, is required to limit the expression of valve margin identify genes including *SHATTERPROOF1 (SHP1), SHATTERPROOF2 (SHP2), ALCATRAZ (ALC)* and *INDEHISCENT (IND)* to the valve edge. On the other hand, expression of the same valve margin genes is limited by the activity of a replum factor (REPLUMLES, RPL), which restricts valve margin differentiation to the valve/replum boundary. We hypothesized that the gene responsible for the *ggl* mutant phenotype might take part in patterning the silique and would therefore be associated with the activities of the factors described above.

To test whether the ectopic differentiation of valve margin-like cells in *ggl* required the activity of *SHP1* and *SHP2* genes we constructed the *shp1*/*shp2*/*ggl* triple mutant. In mature siliques (stage 17) of the *shp1*/*shp2* double mutant lignification of cells in the valve margin adjacent to the dehiscence zone is reduced. Staining of *shp1*/*shp2*/*ggl* siliques with phloroglucinol showed that they also lack lignification in the valve margins (Figure 3), thus suggesting that *shp1* and *shp2* are needed for generating the "lignification bridge" detected in *ggl.* In addition, SEM analysis of the triple *shp1*/*shp2*/*ggl* mutant showed that replum formation is restored to *ggl* siliques when *SHP1* and *SHP2* are non-functional (Figure 5). To further support the evidence that *SHATTERPROOF* genes are ectopically expressed in the *ggl* mutant replum we analyzed the expression of SHP1::*GUS* in the *ggl* mutant background. While in wild type siliques SHP1::*GUS* is expressed precisely in the valve margins, it is ectopically expressed in the replum of the *ggl* mutant. The results provide evidence that ectopic expression of the *GGL* gene results in ectopic expression of *SHATTERPROOF* genes, which therefore contribute to the formation of valve margin-like cells in the replum of *ggl* mutant siliques.

In mature siliques of *ful* mutants valves fail to elongate after fertilization, ectopic lignification occurs in the mesocarp and most notably the replum is enlarged and shows a zigzag pattern in its epidermis. Since replum differentiation in *ggl* mutants is altered we wanted to examine whether this will effect the development of the typical *ful* mutant replum. Indeed, SEM analysis showed that replum development in some siliques was nearly fully restored while in others it was partially restored (Figure 6).

### Example 5 - A Single Zinc-Finger Transcription Factor Family is responsible for ggl Mutant Phenotype

DNA gel blot analysis showed that ggl contains a single transposon insertion. Isolation and sequence analysis of DNA flanking the insertion site further indicated that the insertion is located in an intergenic region on chromosome 1. As can be seen in Figure 7 the location of the 35S enhancer tetramer is between a gene encoding an exostosin family protein with no assigned function (At1g68470; 4718 base pairs upstream of the promoter) and a gene encoding a putative zinc-finger (C2H2) family protein (At1g68480; 3921 base pairs downstream of the promoter). To examine if these two genes were induced in expression in *ggl* compared to wild type, we conducted a Reverse Transcription PCR (RT-PCR) experiment using cDNA isolated from *ggl* and wild type leaf tissues. The results showed that the genes from both sides of the 35S enhancer tetramer were induced in the *ggl* mutant leaves compared to wild type leaves (Figure 8). Subsequent experiments (described below) confirmed that the gene putatively encoding the zinc finger protein *(GGL1)* is responsible for the *ggl* mutant phenotype.

The putative GGL1 protein contains the ERF-associated amphiphlic repression (EAR)-like motif in its amino terminus (Figure 9). The EAR motif has been detected in other transcription factors (Ohta et al., 2001) including the zinc-finger protein SUPERMAN (SUP), which is the closest characterized homolog of GGL1. The GGL1 protein also contains a proline rich motif in its middle part and a putative nuclear localization signal in the amino terminus. Extensive similarity exists in the functional domains described above between GGL1 and another Arabidopsis protein (GGL2) which is shorter (207 amino acids compared to 253 in GGL1) and shows 53% identify to GGL1 at the amino acid level (Figure 11).

### Example 6 - Transgenic plants overexpressing GGL

### Expression of GGL1 under the control of the CαMV35S promoter in Arabidopsis

We expressed the *GGL1* coding region under the control of the *CαMV35S* promoter in transgenic plants. Green, non bleached T0 seedlings which germinated on kanamycin selection media all showed a variety of cotyledon fusion phenotypes and the complete cessation of meristem formation. Root formation in these seedlings did not seem to be affected. In order to obtain fully matured plants we used hypocotyl segments derived from the 35S::*GGL1* seedlings for regeneration experiments. Out of eight mature plants regenerated three showed severe fusion of their side shoots to the main inflorescence stem. Apart from fusions, other phenotypic alterations could not be detected. Seedlings derived from the *35S::GGL1* regenerated plants showed the typical cup shape cotyledon (CUC) phenotypes normally detected in double *cuc* mutants *(Aida et al,* 1999 Development, 126: 1563-70).

### Expression of GGL1 with the 35S enhancer tetramer and its upstream region in Arabidopsis

In order to obtain a more subtle expression of the *GGL1* gene in *Arabidopsis* we expressed its coding region under the control of the 35S enhancer tetramer (used to generate the activation tagging population) which was coupled to a 2kb region upstream of the ATG start codon. Transgenic plants raised in both Columbia and the Ws ecotypes showed to various degrees resemblance to the *ggl* activation tag mutant. The majority of the lines showed the clear effect on silique orientation and pedicel length, while others were even more similar to *ggl,* showing the drop-like rosette leaves (lack of petiole), twisted stem, down pointing siliques and short pedicels, long and twisted cauline leaves and occasionally bracts formation. The latter type of transformants showed a more severe alteration in mature siliques compared to the original *ggl* mutant and alterations to cotyledon shape as they were longer and had a leaf-like shape.

### GGL2 Shows Similar Phenotypes to that Obtained with GGL1

Transgenic plants overexpressing the *GGL2* gene under the control of the 35S CaMV promoter were generated and investigated for alteration in phenotype. Three main classes of phenotypes were observed.

Class 1 plants, showed the most mild effect, their rosette and cauline leaves were slightly revolute, often jagged and were darker green. Flower size in these lines were larger (mainly petals) and they were often semi-sterile. The general appearance of the inflorescence was different from the wild type being more compact, with more flowers. Carpel development was strongly inhibited as siliques were often less than half the size of normal wild type plant siliques. In addition, part of the siliques were pointing down and had shorter pedicels. In rare cases siliques did not shatter, due to what appeared to be ectopic lignification in the replum. Thus, plants overexpressing the GGL2, as those overexpressing GGL1 (see above) could also alter the process of silique shattering and generate non-dehiscent siliques.

A second class of phenotypes were plants which showed the same overall phenotypic alterations as seen in class 1 with the only difference of being retarded in growth and thus half the height of the class 1 plants. The third class were plants which were very severely effected. They were very similar in appearance to the GGL1 plants in the rosette stages, specifically in leaf shape and size. However these plant developed only leafy shoots and thus did not flower. Other plants from this group showed an even stronger phenotype, either being strongly fasciated in the stem or showing strong fusion of the first pair of rosette leaves. In the latter cases often side branches emerged which gave the plants a bushy appearance. In all three classes of phenotypes, phyllotaxy was very strongly effected and flowers or siliques developed in what appeared to a random appearance along the stem and around it.

### Induced expression of GGL1 using the hormone binding domain of the glucocorticoid receptor in Arabidopsis

To overcome the barrier of generating mature *35S::GGL1* plants and examine the consequences of induced ectopic *GGL1* expression in various stages of Arabidopsis plant development we generated transgenic lines which express a fusion protein of *GGL1* and the hormone binding domain of the rat glucocorticoid receptor *(GR;* under the control of the *CαMV35S* promoter). Seeds of *35S::GGL1-GR* primary transformants (T1) were sown on plates containing media with or without 10µM dexamethasone (DEX) hormone. Seedlings sown in media supplemented with DEX showed a clear alteration to hypocotyl and cotyledon morphology compared to those lacking it, which appeared normal. Two major classes of phenotypes could be detected: seedlings in which the cotyledons edges curved upwards (spoon-like shape) and elongated (either with one of the cotyledons pointed up and the other down, or both pointing upwards or both pointing downwards) or a second class in which cotyledons were at the same level and they were strongly misshapen and elongated in a similar manner to the one observed in strong transgenic line overexpressing *GGL1* using the 35S enhancer. In the latter class often one cotyledon was short and the other long. In both classes, hypocotyls were bent downwards, partially touching the media surface. Wild type seedlings grown on DEX hormone containing media did not show this phenotypes. The effect of *GGL1* expression on cotyledon and hypocotyl morphology was increased in a DEX hormone dose dependent manner.

To examine the effect of *GGL1* expression in a later stage of plant development we induced it by spraying soil grown seedlings of the *355:GGL1-*GR transformants with 10µM DEX at different stages of development (a single application at a specific stage) and observed them during the course of development. Seedlings sprayed at early stage (stage A, 9 days from germination, cotyledons fully opened), were strongly affected in their cotyledons which were elongated, and resembled a bottle gourd shape. Spraying at the same stage and at one stage later when two first rosette leaves have developed (stage B, 12 days from germination) resulted in seedlings which were similar to seedlings of the *ggl* mutant with epinastic rosette leaves which lack or had short petioles. Interestingly, spraying plants prior to bolting (Stage C, 21 days from germination, 8 mature rosette leaves) and after bolting but before flowers had opened (Stage D, 26 days from germination) resulted in upwards curling of the leaves, and in more severe cases leaves were even curved inward. Plants sprayed at stage (D) were also effect in their siliques since they were shorter and malformed.

The effect of increased concentrations of DEX hormone were also tested in vivo by spraying nine days old seedlings (cotyledons fully open) once a week (for 4 weeks with 0, 0.001, 0.01, 0.1, 1, and 10µM DEX hormone). Rosette leaves of plants were observed for the upward curling of the leaves phenotypes when they were 5 weeks old. As in the case of the *in vitro* test (see above) the appearance of the phenotype was hormone dose dependent and could be detected in seedlings treated only with 0.1µM up to those treated with 10µM in which the phenotype was most severe and buckled and inwardly curved leaves were detected. Rosette leaves of plants sprayed with solution excluding DEX did not show a change in leaf blade curvature.

### Expression of a dominant negative form of GGL1 under the control of the CαMV35S promoter in Arabidopsis

The putative *GGL1* protein contains an EAR-like motif at its amino-terminus, which renders it a transcriptional repressor. To examine the outcome of forming a negative dominant allele, we constructed transgenic lines which overexpress a truncated GGL1 protein lacking the EAR domain (35S::*GGL1*-EAR⁻). Out of seventeen kanamycin positive seedlings seven gave rise to plants with curled leaves. Both rosette and mainly cauline leaves showed curling upwards of blades compared to wild type leaves. Other lateral organs did not show a phenotypic effect. The overall 35S::*GGL1*-EAR⁻ plants stature was smaller compared to wild type. This change in phenotype indicates that the EAR-like domain has a function in the GGL1 protein, supporting the view of GGL proteins being transcriptional repressors.

### Expression of GGL1 under the control of the CαMV35S promoter in Tobacco

The effect of *GGL1* overexpression on hetorologous plant development was tested in tobacco (cv. Samsun). Apart from causing strong dwarfism, overexpression of *GGL1* (using the 35S::*GGL1* vector, see above) resulted in major effects on both vegetative and reproductive organs in all 18 transgenic plants generated. The phenotype in leaves was intriguing since it closely resembled the leaf curvature defects obtained by overexpressing the *GGL1* dominant negative form in Arabidopsis (35S::*GGL1*-EAR⁻ lines, see above) and also (to less extent) the late induction phenotype of *GGL1* in the 35S::*GGL1-GR* transgenic lines. Compared to wild type tobacco leaves, those of 35S::*GGL1* lines were smaller, lacked or comprised only a very short petiole and their blade margins were inwardly curved. In other cases leaves had an involute appearance or otherwise (in lines showing a mild phenotype) the blade was crinkly and buckled. In all cases, leaves of the 35S::*GGL1* lines showed local, adaxial bulging of the lamina in between the veins.

### Example 7 - Overexpression of a Putative Tomato Ortholog Results in Similar Phenotype

We used sequence information derived from a tomato *(Lycopersicon esculentum)* Expressed Sequence Tag (EST247066, tomato ovary, 5 days pre anthesis to 5 days post-anthesis cDNA library) to clone a full length tomato ortholog of the Arabidopsis *GGL* gene (the tomato gene was termed *LeGGL,* Figure 15). Full-length cDNAs were cloned using the SMART RACE cDNA Amplification Kit (Clontech) according to the manufacturer's instructions, with slight modifications to annealing temperatures (normally reduced by 5 to 10°C relative to recommended temperatures) or numbers of cycles (up to 35 cycles). 3'RACE-PCR was conducted using the AP79 and AP80 (nested) oligonucleotides:

AP79- 5' AACTCCAACTATAGCCCTC 3'

AP80- 5' CGGATCCACTAGTAGTCATGAGTCCAG

The entire *LeGGL* cDNA is 1163 bp long, and it encodes a protein of 259 amino acid residues.

Oligonucleotides AP84 and AP80 were used to amplify *LeGGL* and introduced BamHI and XhoI restriction sites to the amplified fragment at the 5' and 3', which were utilized to ligate the coding region fragment to the BamHI and SalI sites in a modified pNEW binary vector (van Engelen et al, 1995, supra) in between the 35S CaMV promoter and the NOS terminator. Transgenic plants overexpressing the *LeGGL* showed the typical cup shape cotyledon *(cuc)* phenotype as detected in transgenic seedlings overexpressing the *Arabidopsis GGL1* under the 35S CaMV promoter.

AP84- 5' CCTCGAGTTTCTCTACGTTGTCAGCG 3'

AP85- 5'GGAGGAGGTTACCACCAATCA 3

### Example 8 - Spatial and Temporal Expression of GGL1

To analyze *GGL1* gene expression we constructed two different *GGL1*-Promoter::*GUS* (β-Glucuronidase) fusion constructs. pGGL1::*GUS* contains the 2kb genomic DNA upstream of the putative transcription start site fused to the uidA gene, while pGGL1plus::*GUS* contained a longer fragment fused to the uidA gene which included the same 2kb genomic fragment plus the first exon and intron of the *GGL1* gene. We detected GUS activity in young seedlings and flowers (mainly the developing carpel) in transgenic lines generated with the two constructs described above, in both cases GUS staining was comparable (Figure 10).

### Example 9 - Composition change affected by ectopic lignification by GGL overexpression

Fluorescence spectroscopy is one of the most sensitive spectroscopic methods to characterize different chemical compounds or similar compounds within a different chemical (electronic) environment. Using an excitation-emission scan, fluorescence spectroscopy can even spectroscopically differentiate mixed components. There is little probability that different chemical compounds show the same excitation and/or emission spectrum. Similarly, identical molecules may have a peak shift in their excitation or emission spectrum when they are embedded in a different environment e.g. attached to a cell wall or a "free'' molecule in solution. But due to the many possibilities which may occur, it is always difficult to attribute the resulting spectral shifts and differences to specific compounds. However, when the spectral information is used as a fingerprint information, it can locate major changes e.g. in a plant. Combined with other methods it will provide a deeper insight into molecular markers and their changes during development. We used this technique for fingerprinting the wild type and *ggl* siliques.

The fluorescence of a cross-section of *ggl* and wild type mature siliques is shown in Figure 12A together with the positions where the laterally resolved spectra has been measured. Each of the 14 measurement points (5-10 microns) was excited at a wavelength of 360 nm and the auto fluorescence emission spectra for each location is shown in Figure 12B. We used Multivariate Curve Resolution (MCR) to deconvolute the spectra by means of a Principle Component Analysis (PCA). MCR deconvolutes the spectra into two factors which are shown in Figure 12C. The two factors explain 94 % of the variance of the spectra. Factor 12D shows a maximum at around 470 nm (together with a high contribution in the blue region), factor 2 a maximum at around 515 nm. These two factors may be attributed to different lignin components. It is well known, that there are G, GS and GSH type of lignin in wood (guaiacil, syringyl and 4-hydroxiohenyl - units). The major difference is their CH3O-content of the basic units. Most probably, the two factors relate to a more "hardwood like" lignin structure and to a more "softwood like" lignin structure. The relative concentrations of either one of the two factors for each location measured from a cross section of *ggl* and wild type is shown in Figure 12D. As can be seen from the plot, factor 1 contributes mainly to the spectra in the wild type plant, whereas in the *ggl* type, both factors are almost equally distributed at each location. In addition factor 1 takes prominence in locations 2 and 3 where a lignification bridge has been observed (Figure 3B). The other positions of the *ggl* mutant show clear reduction of the wild-type component (factor 1), signifying a change in the major lignification components.

## Claims

1. Method to prevent dehiscence in a plant comprising providing said plant with a nucleotide protein coding for a GGL protein or a functional equivalent thereof, and growing said plant.

2. Method according to claim 1, wherein the GGL protein is overexpressed in the replum of a plant.

3. Method according to claim 2, wherein said plant is a member of the *Brassicaceae,* preferably *Brassica napus.*

4. Method to alter lignification in a plant comprising providing said plant with a nucleotide protein coding for a GGL protein or a functional equivalent thereof and growing said plant.

5. A GGL protein having a sequence comprising the amino acid sequence of Fig. 13B, 14B or 15B, or a functional fragment thereof.

6. A nucleotide sequence coding for a GGL protein according to claim 5, preferably comprising the nucleotide sequence of Fig. 13A, 14A or 15A.

7. A vector comprising the nucleotide sequence of claim 6.

8. A vector according to claim 7, wherein the nucleotide sequence of claim 6 is operably linked with a replum specific promoter.

9. A host cell comprising a vector according to claim 7 or 8.

10. A plant transformed with a vector according to claim 7 or 8, or by a host cell according to claim 9.

11. Use of a GGL protein according to claim 5, a nucleotide sequence according to claim 6, a vector according to claim 7 or 8, or a host cell according to claim 9 to perform a method according to any of claims 1-4.


### Annex to the application documents - subsequently filed sequences listing

SEQUENCE LISTING

<110> Plant Research International B.V.

<120> Method for preventing dehiscence and altering plant lignification

<130> P67264EP00

<140> EP 04077174.3
<141> 2004-07-28

<160> 28

<170> PatentIn version 3.1

<210> 1
<211> 7
<212> PRT
<213> Arabidopsis thaliana

<220>
<221> SITE
<222> (1)..(7)
<223> EAR motif

<400> 1
Leu Asp Leu Asn Asn Leu Pro
1               5

<210> 2
<211> 7
<212> PRT
<213> Arabidopsis thaliana

<220>

<221>  SITE

<222>  (1)..(7)

<223>  Zinc finger motif

<400>  2

Lys Ser Gln Ala Leu Gly Gly
1               5

<210>  3

<211>  6

<212>  PRT

<213>  Arabidopsis thaliana

<220>

<221>  SITE

<222>  (1)..(6)

<223>  Zinc finger motif

<400>  3

Gln Ala Leu Gly Gly His
1               5

<210>  4

<211>  7

<212>  PRT

<213>  Arabidopsis thaliana

<220>

<221>  SITE

<222>  (1)..(7)

<223>  EAR motif

<220>

<221>  MISC_FEATURE

<222>  (1)..(1)

<223>  "X" stands for L/F

<220>

<221>  MISC_FEATURE

<222>  (5)..(5)

<223>  "X" stands for L/F


<220>

<221>  MISC_FEATURE

<222>  (6)..(6)

<223>  "X" is any amino acid


<400>  4

Xaa Asp Leu Asn Xaa Xaa Pro
1               5


<210>  5

<211>  27

<212>  DNA

<213>  Artificial Sequence


<220>

<223>  Oligonucleotide AP55

<400>  5
cggatccttc ccactatgag gcatgag                                          27


<210>  6

<211>  27

<212>  DNA

<213>  Artificial Sequence


<220>

<223>  Oligonucleotide AP44

<400>  6
cgagctctca gagcgagtga tgatctt                                          27


<210>  7

<211>  25

<212>  DNA

<213>  Artificial Sequence

<220>

<223>  Oligonucleotide AP57

<400>  7
cggatccaat gacggaggta cgttg                                          25

<210>  8

<211>  28

<212>  DNA

<213>  Artificial Sequence

<220>

<223>  Oligonucleotide AP58

<400>  8
cgagctctta tagcccatga tgtggagg                                       28

<210>  9

<211>  29

<212>  DNA

<213>  Artificial Sequence

<220>

<223>  Oligonucleotide AP53

<400>  9
Caagcttgct tccgtgagag tgaattgtg                                      29

<210>  10

<211>  28

<212>  DNA

<213>  Artificial Sequence

<220>

<223>  Oligonucleotide AP54

<400>  10
cccatgggtg ggaaatgaga gattggcg                                       28

<210> 11
<211> 30
<212> DNA
<213> Artificial Sequence

<220>
<223> Oligonucleotide AP67
<400> 11
cccatgggta attctcctca tgcctcctgc 30

<210> 12
<211> 22
<212> DNA
<213> Artificial Sequence

<220>
<223> Primer RACTP1
<400> 12
gcggttttcc ccagtgttgt tg 22

<210> 13
<211> 24
<212> DNA
<213> Artificial Sequence

<220>
<223> Primer RACTP2
<400> 13
tgcctggacc tgcttcatca tact 24

<210> 14
<211> 27
<212> DNA
<213> Artificial Sequence

<220>
<223> Oligonucleotide AP43

<400>  14
cggatccatg atatatcctg gaagctc                                          27

<210>  15

<211>  28

<212>  DNA

<213>  Artificial Sequence

<220>

<223>  Oligonucleotide AP45

<400>  15
cggatcccca tgactttcaa taagaggc                                         28

<210>  16

<211>  29

<212>  DNA

<213>  Artificial Sequence

<220>

<223>  Oligonucleotide AP46

<400>  16
cgtcgacgag tagcacaaaa ctataggtc                                        29

<210>  17

<211>  20

<212>  DNA

<213>  Artificial Sequence

<220>

<223>  Oligonucleotide AP79

<400>  17
aactccaact atagcccctc                                                  20

<210>  18

<211>  27

<212>  DNA

<213>  Artificial Sequence

<220>

<223>  Oligonucleotide AP80

<400>  18
cggatccact agtagtcatg agtccag                                          27

<210>  19

<211>  26

<212>  DNA

<213>  Artificial Sequence

<220>

<223>  Oligonucleotide AP84

<400>  19
cctcgagttt ctctacgttg tcagcg                                           26

<210>  20

<211>  21

<212>  DNA

<213>  Artificial Sequence

<220>

<223>  Oligonucleotide AP85

<400>  20
ggaggaggtt accaccaatc a                                                21

<210>  21

<211>  253

<212>  PRT

<213>  Arabidopsis thaliana

<400>  21

Met Pro His Glu Glu Asn Tyr Leu Asp Leu Asn Asn Leu Pro Asp Asp
1               5                   10                  15

Phe Ser Lys Asp Gly Asn Lys Gln Ala Leu Glu Glu Gly Ser Ser Ser
            20                  25                  30

Gly Gln Arg Lys Lys Lys Gly Ser Lys Glu Gly Lys Asp Glu Ser Gly
        35                  40                  45

Lys Val Tyr Glu Cys Arg Phe Cys Ser Leu Lys Phe Cys Lys Ser Gln
50                  55                  60

Ala Leu Gly Gly His Met Asn Arg His Arg Gln Glu Arg Glu Thr Glu
65                  70                  75                  80

Thr Leu Asn Gln Ala Arg Gln Leu Val Tyr Arg Asn Asp Thr Ile Thr
                85                  90                  95

Pro Pro Gly Ile Ser Pro Phe Gly Tyr His His Thr Thr Asp Pro Thr
            100                 105                 110

Ile Tyr Arg Ser Val Tyr Ser Ser Pro Met Ile Tyr Pro Gly Ser Ser
        115                 120                 125

Ser Thr Asn Leu Val Pro Gln Pro Pro Met Pro Pro Pro Pro Pro Pro
    130                 135                 140

Tyr Pro Tyr Ser Ser Asn Gln Tyr Ser Pro His Asn His Phe Asn Asp
145                 150                 155                 160

Tyr Tyr Leu Asn Pro Ser Phe Arg Gly Ser Arg Ser Ile Ser Pro Ser
                165                 170                 175

Pro Asn Leu Pro Thr Thr Thr Thr Val Asp Tyr Met Ala Asp Ser Pro
            180                 185                 190

Val Glu Pro Gly Tyr Thr Cys Val Gly Ala Pro Ile Gly Pro Thr Gly
        195                 200                 205

Phe Pro Ile Arg Gly Pro Ser Ile Val Arg Ala Pro Leu Glu Pro Pro
    210                 215                 220

Gln Gly Arg Asp Gly Asp Ala Ser Arg Gln Arg Leu Asp His Ser Leu
225                 230                 235                 240

Arg Phe Pro Ile Asn Arg Phe Gln Asp His His Ser Leu
                245                 250

<210>  22

<211>  263

<212>  PRT

<213>  Oryza sativa


<400>  22

Met Asn Ser Ser Arg Arg Gln Glu Gly Ser Pro Leu Asp Leu Asn Asn
1               5                   10                  15

Leu Pro Asp Glu Phe Gly Lys Gln Thr Val Glu Ser Ser Thr Thr Thr
            20                  25                  30

Ala Ala Ser Ser Ala Glu Ala Ser Arg Val Thr Lys Lys Lys Ser Asn
        35                  40                  45

Gly Gly Lys Asp Glu Ala Gly Lys Val Tyr Glu Cys Arg Phe Cys Ser
    50                  55                  60

Leu Lys Phe Cys Lys Ser Gln Ala Leu Gly Gly His Met Asn Arg His
65                  70                  75                  80

Arg Gln Glu Arg Glu Thr Glu Thr Leu Asn Arg Ala Arg Gln Leu Val
                85                  90                  95

Phe Gly Asn Asp Ser Leu Ala Ala Val Gly Ala Gln Leu Asn Phe Arg
            100                 105                 110

Asp Val Asn Met Gly Gly Gly Gly Ala Ala Ala Pro Pro Pro Thr Met
        115                 120                 125

Gln Met Gly Gly Gly Gly Phe Arg Gly Gly Gly Val Gly Gly Asp Pro
    130                 135                 140

Cys Ile Pro Leu Arg Pro Val Gln Pro Arg Leu Ser Pro Pro Gln Pro
145                 150                 155                 160

Pro Pro Tyr His His Tyr Leu Tyr Thr Thr Thr Ala Pro Pro Ser Ala
                165                 170                 175

Leu His Pro Met Ser Tyr Pro Ala Thr Tyr Pro Ala Pro Pro Arg His
            180                 185                 190

Gln Gln Pro Ala Ala Val Gly Asp Tyr Val Ile Gly His Ala Val Ser
        195                 200                 205

Ala Gly Asp Ala Leu Val Ala Pro Pro Pro Pro Pro His Arg Ala Ser
    210                 215                 220

Phe Ser Cys Phe Gly Ala Pro Leu Ala Ala Pro Pro Ala Asn Val Gln
225                 230                 235                 240

Pro Asp Asn Gly Asn Cys Asn Cys Ser Phe Gly Cys Gly His Ser Asn
                245                 250                 255

Arg Asn Val Asn Ala Ala Ser
            260

<210> 23

<211> 1224

<212>  DNA

<213>  Arabidopsis thaliana

<220>

<221>  CDS

<222>  (234)..(995)

<223>

<400>  23
   ttagtttcca cacgcagaga gagaaacaaa gagagagatg ggagggaggg tttgtggcag      60
   ataaactgat agaaaacttt cgaacctctc ttctttaact tttactactc tctattcgtt     120
   tttctcttcc ttttgtggtg ccgcctccaa ctgaaagtct tctaatatat attctcaatt     180
   attctagctc tccgggagaa gtcttatctc acgccaatct ctcatttccc act atg        236
                                                              Met
                                                              1
   agg cat gag gag aat tac tta gac ctc aac aat ctt cca gat gat ttt       284
   Arg His Glu Glu Asn Tyr Leu Asp Leu Asn Asn Leu Pro Asp Asp Phe
               5                   10                  15
   tct aaa gat ggc aat aag caa gcc ctc gag gaa ggt tct tcc tct ggt       332
   Ser Lys Asp Gly Asn Lys Gln Ala Leu Glu Glu Gly Ser Ser Ser Gly
           20                  25                  30
   caa aga aag aag aaa ggg agt aaa gaa ggg aaa gat gag agt ggg aaa       380
   Gln Arg Lys Lys Lys Gly Ser Lys Glu Gly Lys Asp Glu Ser Gly Lys
       35                  40                  45
   gtg tac gaa tgt cga ttt tgt tca ctc aag ttc tgc aaa tcc caa gct       428
   Val Tyr Glu Cys Arg Phe Cys Ser Leu Lys Phe Cys Lys Ser Gln Ala
   50                  55                  60                  65
   ctt ggt ggc cac atg aat cgc cac cga caa gag agg gag acc gag aca       476
   Leu Gly Gly His Met Asn Arg His Arg Gln Glu Arg Glu Thr Glu Thr
                   70                  75                  80
   ttg aat caa gct cgt caa ttg gtc tac cgt aac gat acc ata acc cca       524
   Leu Asn Gln Ala Arg Gln Leu Val Tyr Arg Asn Asp Thr Ile Thr Pro
               85                  90                  95
   cct gga att tct cct ttc ggc tat cat cat acc aca gat cca aca ata       572
   Pro Gly Ile Ser Pro Phe Gly Tyr His His Thr Thr Asp Pro Thr Ile
           100                 105                 110
   tac cgg tcg gtt tac tca tca cca atg ata tat cct gga agc tct tca       620
   Tyr Arg Ser Val Tyr Ser Ser Pro Met Ile Tyr Pro Gly Ser Ser Ser
       115                 120                 125
   acg aac ttg gtt ccg caa ccg ccg atg cca cca cca ccg cct cca tat       668
   Thr Asn Leu Val Pro Gln Pro Pro Met Pro Pro Pro Pro Pro Pro Tyr
   130                 135                 140                 145
   cca tac tct tca aac caa tac tct cct cac aac cac ttc aac gac tac       716
   Pro Tyr Ser Ser Asn Gln Tyr Ser Pro His Asn His Phe Asn Asp Tyr
                   150                 155                 160
   tac tta aac cca tct ttt aga ggc agc aga agc atc tct cca agt cct       764
   Tyr Leu Asn Pro Ser Phe Arg Gly Ser Arg Ser Ile Ser Pro Ser Pro
               165                 170                 175
   aac ctc ccc acc acc acc acc gtt gat tac atg gcc gat agt ccc gtg      812
   Asn Leu Pro Thr Thr Thr Thr Val Asp Tyr Met Ala Asp Ser Pro Val
           180                 185                 190
   gaa ccg ggc tac act tgt gtt ggt gca ccc att ggt ccg acc ggt ttc      860
   Glu Pro Gly Tyr Thr Cys Val Gly Ala Pro Ile Gly Pro Thr Gly Phe
       195                 200                 205
   cct atc cgt ggc cca agc atc gtc cgt gcg ccg ctt gaa cca cca caa      908
   Pro Ile Arg Gly Pro Ser Ile Val Arg Ala Pro Leu Glu Pro Pro Gln
   210                 215                 220                 225
   ggt cgt gat ggt gac gct tca cga cag cgt ttg gat cac tct ctt agg      956
   Gly Arg Asp Gly Asp Ala Ser Arg Gln Arg Leu Asp His Ser Leu Arg
                   230                 235                 240
   ttc ccc atc aat cgg ttt caa gat cat cac tcg ctc tga gttttaatag      1005
   Phe Pro Ile Asn Arg Phe Gln Asp His His Ser Leu
               245                 250
   attctctacg cttttctttt cttttctttt tttgtttttt tttgggtatt ttctcgtcct      1065
   cttgttttgt aactttgttg gttctatcta aaagtagaga tctagaattg tctaaacgcc      1125
   aagatcctac atgtgttatt atcataatga catgtttgtt ggtttttatt ggtctagtcg      1185
   aatactctaa ttattcacaa ccggattttg ttttatgtc                             1224

<210>  24

<211>  253

<212>  PRT

<213>  Arabidopsis thaliana

<400>  24

Met Arg His Glu Glu Asn Tyr Leu Asp Leu Asn Asn Leu Pro Asp Asp
1               5                   10                  15

Phe Ser Lys Asp Gly Asn Lys Gln Ala Leu Glu Glu Gly Ser Ser Ser
            20                  25                  30

Gly Gln Arg Lys Lys Lys Gly Ser Lys Glu Gly Lys Asp Glu Ser Gly
        35                  40                  45

Lys Val Tyr Glu Cys Arg Phe Cys Ser Leu Lys Phe Cys Lys Ser Gln
    50                  55                  60

Ala Leu Gly Gly His Met Asn Arg His Arg Gln Glu Arg Glu Thr Glu
65                  70                  75                  80

Thr Leu Asn Gln Ala Arg Gln Leu Val Tyr Arg Asn Asp Thr Ile Thr
                85                  90                  95

Pro Pro Gly Ile Ser Pro Phe Gly Tyr His His Thr Thr Asp Pro Thr
            100                 105                 110

Ile Tyr Arg Ser Val Tyr Ser Ser Pro Met Ile Tyr Pro Gly Ser Ser
        115                 120                 125

Ser Thr Asn Leu Val Pro Gln Pro Pro Met Pro Pro Pro Pro Pro Pro
    130                 135                 140

Tyr Pro Tyr Ser Ser Asn Gln Tyr Ser Pro His Asn His Phe Asn Asp
145                 150                 155                 160

Tyr Tyr Leu Asn Pro Ser Phe Arg Gly Ser Arg Ser Ile Ser Pro Ser
                165                 170                 175

Pro Asn Leu Pro Thr Thr Thr Thr Val Asp Tyr Met Ala Asp Ser Pro
            180                 185                 190

Val Glu Pro Gly Tyr Thr Cys Val Gly Ala Pro Ile Gly Pro Thr Gly
        195                 200                 205

Phe Pro Ile Arg Gly Pro Ser Ile Val Arg Ala Pro Leu Glu Pro Pro
    210                 215                 220

Gln Gly Arg Asp Gly Asp Ala Ser Arg Gln Arg Leu Asp His Ser Leu
225                 230                 235                 240

Arg Phe Pro Ile Asn Arg Phe Gln Asp His His Ser Leu
                245                 250

<210> 25

<211> 781

<212> DNA

<213> Arabidopsis thaliana

<220>

<221> CDS

<222> (158)..(781)

<223>

<400> 25
gagttccatg ctcaatgttc tcatttctaa agagataaaa aagagagaga taaactgatt 60

aaaagaaatt tcacaaagct ctctttaatt tatccctcca tcttcactct ctcaactctt 120

tatccttagt ttcgtctctc ctgtcttctt ggtaacc atg aga gct gat gaa aat 175
Met Arg Ala Asp Glu Asn
1               5

aac act tta gat ctt aat aac tta cct gat gat cct tct aga gac att 223
Asn Thr Leu Asp Leu Asn Asn Leu Pro Asp Asp Pro Ser Arg Asp Ile
            10                  15                  20

ttc cca ttc ttt gaa gaa ggc ttc tct tca tct tct tct tct gga ggg 271
Phe Pro Phe Phe Glu Glu Gly Phe Ser Ser Ser Ser Ser Ser Gly Gly
        25                  30                  35

ttt aga gag aag caa aca aaa gat ggg aaa gag tat gaa tgt aga ttt 319
Phe Arg Glu Lys Gln Thr Lys Asp Gly Lys Glu Tyr Glu Cys Arg Phe
    40                  45                  50

tgt tca cta aag ttc ttc aaa tct caa gct ctc ggt ggc cac atg aac 367
Cys Ser Leu Lys Phe Phe Lys Ser Gln Ala Leu Gly Gly His Met Asn
55                  60                  65                  70

cgc cac cgt caa gaa aga gag acg gag tca cta aac aaa gct cgt gaa 415
Arg His Arg Gln Glu Arg Glu Thr Glu Ser Leu Asn Lys Ala Arg Glu
                75                  80                  85

ctt gtt ctt cgc aac gat agc ttt cct cct cat caa gga cct cca tcg 463
Leu Val Leu Arg Asn Asp Ser Phe Pro Pro His Gln Gly Pro Pro Ser
            90                  95                  100

ttt agt tat cat caa gga gat gtg cat ata gga gac cta aca caa ttt 511
Phe Ser Tyr His Gln Gly Asp Val His Ile Gly Asp Leu Thr Gln Phe
        105                 110                 115

aaa cca atg atg tac cca ccg aga cat ttc tca ctc ccc ggc tct tcc 559
Lys Pro Met Met Tyr Pro Pro Arg His Phe Ser Leu Pro Gly Ser Ser
    120                 125                 130

tcc atc ctc cag ctg caa cca cca tat ctt tat cct ccg ctg tct tca 607
Ser Ile Leu Gln Leu Gln Pro Pro Tyr Leu Tyr Pro Pro Leu Ser Ser
135                 140                 145                 150

ccg ttt cct caa cat aac acc aac ata ggc aac aac ggt acg cgt cac 655
Pro Phe Pro Gln His Asn Thr Asn Ile Gly Asn Asn Gly Thr Arg His
                155                 160                 165

caa acc cta acc aat agc gtt tgc ggt ggt cgt gca cta cct gac tca 703
Gln Thr Leu Thr Asn Ser Val Cys Gly Gly Arg Ala Leu Pro Asp Ser
            170                 175                 180

agt tac acc ttc atc ggt gca cca gtg gct aat ggt tct aga gtt gct 751
Ser Tyr Thr Phe Ile Gly Ala Pro Val Ala Asn Gly Ser Arg Val Ala
        185                 190                 195

cca cat cta cct cca cat cat ggg cta taa 781
Pro His Leu Pro Pro His His Gly Leu
    200                 205

<210> 26

<211> 207

<212> PRT

<213> Arabidopsis thaliana

<400> 26

Met Arg Ala Asp Glu Asn Asn Thr Leu Asp Leu Asn Asn Leu Pro Asp
1               5                   10                  15

Asp Pro Ser Arg Asp Ile Phe Pro Phe Phe Glu Glu Gly Phe Ser Ser
            20                  25                  30

Ser Ser Ser Ser Gly Gly Phe Arg Glu Lys Gln Thr Lys Asp Gly Lys
        35                  40                  45

Glu Tyr Glu Cys Arg Phe Cys Ser Leu Lys Phe Phe Lys Ser Gln Ala
    50                  55                  60

Leu Gly Gly His Met Asn Arg His Arg Gln Glu Arg Glu Thr Glu Ser
65                  70                  75                  80

Leu Asn Lys Ala Arg Glu Leu Val Leu Arg Asn Asp Ser Phe Pro Pro
                85                  90                  95

His Gln Gly Pro Pro Ser Phe Ser Tyr His Gln Gly Asp Val His Ile
            100                 105                 110

Gly Asp Leu Thr Gln Phe Lys Pro Met Met Tyr Pro Pro Arg His Phe
        115                 120                 125

Ser Leu Pro Gly Ser Ser Ser Ile Leu Gln Leu Gln Pro Pro Tyr Leu
    130                 135                 140

Tyr Pro Pro Leu Ser Ser Pro Phe Pro Gln His Asn Thr Asn Ile Gly
145                 150                 155                 160

Asn Asn Gly Thr Arg His Gln Thr Leu Thr Asn Ser Val Cys Gly Gly
                165                 170                 175

Arg Ala Leu Pro Asp Ser Ser Tyr Thr Phe Ile Gly Ala Pro Val Ala
            180                 185                 190

Asn Gly Ser Arg Val Ala Pro His Leu Pro Pro His His Gly Leu
        195                 200                 205

<210>  27

<211>  1163

<212>  DNA

<213>  Lycopersicon esculentum


<220>

<221>  CDS

<222>  (196)..(975)

<223>

<400> 27
tttagagaga gatttggcag atatatagag agaaagcccc acaaaacacc ctcagccttt 60

ttcttcatca tcactcaaag tctcaaactc aaactcaaac tccaactata gccctctata 120

catttcagtt tcaaactgaa agtcttttta attatttggc caaaacttct ttactcactt 180

ctactactag tagtc atg agt cca gaa aga aat cca ctt gac ctc aac aat 231
Met Ser Pro Glu Arg Asn Pro Leu Asp Leu Asn Asn
1               5                   10

tta cct cat gaa gaa ttt tgt aga gat ggt aaa caa gtc ctt gaa gga 279
Leu Pro His Glu Glu Phe Cys Arg Asp Gly Lys Gln Val Leu Glu Gly
        15                  20                  25

gga ggg tat aga aaa aag aaa aac ggc ggt gaa gaa gat tgt ggt aaa 327
Gly Gly Tyr Arg Lys Lys Lys Asn Gly Gly Glu Glu Asp Cys Gly Lys
    30                  35                  40

gtt tat gag tgt agg ttt tgt tcc ctc aaa ttt tgc aaa tct caa gca 375
Val Tyr Glu Cys Arg Phe Cys Ser Leu Lys Phe Cys Lys Ser Gln Ala
45                  50                  55                  60

ctt ggg gga cac atg aac cgc cac cgt caa gag agg gag aca gaa aca 423
Leu Gly Gly His Met Asn Arg His Arg Gln Glu Arg Glu Thr Glu Thr
                65                  70                  75

cta aat cga gct cgt caa ctg gtg ttc aat aat gat aac ata atc cct 471
Leu Asn Arg Ala Arg Gln Leu Val Phe Asn Asn Asp Asn Ile Ile Pro
            80                  85                  90

cca cat cac ctt agt tgt cct cca att cct cat gga gga ggt tac cac 519
Pro His His Leu Ser Cys Pro Pro Ile Pro His Gly Gly Gly Tyr His
        95                  100                 105

caa tca aca aac att ggt gat cca acg ctc tct tat agg ccg ccg ccg 567
Gln Ser Thr Asn Ile Gly Asp Pro Thr Leu Ser Tyr Arg Pro Pro Pro
    110                 115                 120

cca cca cca cca tca ctt tat cca aca ggg tta ttt tca gga aat tct 615
Pro Pro Pro Pro Ser Leu Tyr Pro Thr Gly Leu Phe Ser Gly Asn Ser
125                 130                 135                 140

acc act act ctc ttg cca cct cca cca aca caa ccg cca cat caa cca 663
Thr Thr Thr Leu Leu Pro Pro Pro Pro Thr Gln Pro Pro His Gln Pro
                145                 150                 155

tca tac atg tac gcc tct cca ccg cgt atg gtt tca ttc tct tct cat 711
Ser Tyr Met Tyr Ala Ser Pro Pro Arg Met Val Ser Phe Ser Ser His
            160                 165                 170

caa tat ccg gct caa aca aat gat tac ttc cta ggc cat gtc ttg tca 759
Gln Tyr Pro Ala Gln Thr Asn Asp Tyr Phe Leu Gly His Val Leu Ser
        175                 180                 185

gaa tcc aac aca cac tcg aca agt act acc aat aac ttc atg gga tca 807
Glu Ser Asn Thr His Ser Thr Ser Thr Thr Asn Asn Phe Met Gly Ser
    190                 195                 200

tcc gct ctg ggg cct gat cat ggt agc aat aac aat aat aac aac aat 855
Ser Ala Leu Gly Pro Asp His Gly Ser Asn Asn Asn Asn Asn Asn Asn
205                 210                 215                 220

aat agt aat aat tac act tgc att ggt gca cca ata ggg cat gga ctt 903
Asn Ser Asn Asn Tyr Thr Cys Ile Gly Ala Pro Ile Gly His Gly Leu
                225                 230                 235

gga ctt ggc aat aat gga agt ggc tct agt ggg aaa caa caa cat ttg     951
Gly Leu Gly Asn Asn Gly Ser Gly Ser Ser Gly Lys Gln Gln His Leu
            240                 245                 250

gat cga tta caa gat gga ttt tag tagtttttg tcattatatt ttgctttatt     1005
Asp Arg Leu Gln Asp Gly Phe
        255

ttcttgttct atgtttaacc ttctttttgt tctagtctta attatctttt taatctagaa     1065

ttataattaa agttttgtac gctgacaacg tagagaaatt ttaccctatg atcatcagtg     1125

taatttaacc aaaaaaaaaa aaaaaaaaaa aaaaaaaa     1163

<210>  28

<211>  259

<212>  PRT

<213>  Lycopersicon esculentum

<400>  28

Met Ser Pro Glu Arg Asn Pro Leu Asp Leu Asn Asn Leu Pro His Glu
1               5                   10                  15

Glu Phe Cys Arg Asp Gly Lys Gln Val Leu Glu Gly Gly Gly Tyr Arg
            20                  25                  30

Lys Lys Lys Asn Gly Gly Glu Glu Asp Cys Gly Lys Val Tyr Glu Cys
        35                  40                  45

Arg Phe Cys Ser Leu Lys Phe Cys Lys Ser Gln Ala Leu Gly Gly His
    50                  55                  60

Met Asn Arg His Arg Gln Glu Arg Glu Thr Glu Thr Leu Asn Arg Ala
65                  70                  75                  80

Arg Gln Leu Val Phe Asn Asn Asp Asn Ile Ile Pro Pro His His Leu
                85                  90                  95

Ser Cys Pro Pro Ile Pro His Gly Gly Gly Tyr His Gln Ser Thr Asn
            100                 105                 110

Ile Gly Asp Pro Thr Leu Ser Tyr Arg Pro Pro Pro Pro Pro Pro Pro
        115                 120                 125

Ser Leu Tyr Pro Thr Gly Leu Phe Ser Gly Asn Ser Thr Thr Thr Leu
    130                 135                 140

Leu Pro Pro Pro Pro Thr Gln Pro Pro His Gln Pro Ser Tyr Met Tyr
145                 150                 155                 160

Ala Ser Pro Pro Arg Met Val Ser Phe Ser Ser His Gln Tyr Pro Ala
                165                 170                 175

Gln Thr Asn Asp Tyr Phe Leu Gly His Val Leu Ser Glu Ser Asn Thr
            180                 185                 190

His Ser Thr Ser Thr Thr Asn Asn Phe Met Gly Ser Ser Ala Leu Gly
        195                 200                 205

Pro Asp His Gly Ser Asn Asn Asn Asn Asn Asn Asn Asn Ser Asn Asn
    210                 215                 220

Tyr Thr Cys Ile Gly Ala Pro Ile Gly His Gly Leu Gly Leu Gly Asn
225                 230                 235                 240

Asn Gly Ser Gly Ser Ser Gly Lys Gln Gln His Leu Asp Arg Leu Gln
                245                 250                 255

Asp Gly Phe